# EUROPÄISCHE PATENTSCHRIFT

(11) **EP 1 996 171 B1**
(45) Veröffentlichungstag und Bekanntmachung des Hinweises auf die Patenterteilung: **17.11.2010**
(21) Anmeldenummer: 07723076.1
(22) Anmeldetag: 07.03.2007
(51) Int. Cl.: A61K 9/70, B65D 75/30

(54) **WIRKSTOFFHALTIGE PFLASTER MIT VERBESSERTER HANDHABUNG**
EASY-TO-HANDLE PLASTER CONTAINING AN ACTIVE SUBSTANCE
PANSEMENTS CONTENANT DES SUBSTANCES ACTIVES CARACTÉRISÉS PAR UN MANIEMENT AMÉLIORÉ

(30) Priorität: 09.03.2006 DE 102006011340
(43) Veröffentlichungstag der Anmeldung: 03.12.2008
(73) Patentinhaber: Grünenthal GmbH, 52078 Aachen (DE)
(72) Erfinder: KUGELMANN, Heinrich, 52068 Aachen (DE); BARTHOLOMÄUS, Johannes, 52080 Aachen (DE)
(74) Vertreter: Bülle, Jan
(86) Internationale Anmeldenummer: PCT/EP2007/001932
(87) Internationale Veröffentlichungsnummer: WO 2007/101659

(56) Entgegenhaltungen:
- EP-A2- 1 103 241
- EP-B1- 0 922 651
- WO-A-95/00122
- US-A- 3 153 868
- US-B1- 6 467 621

## Beschreibung

Die Erfindung betrifft ein System, welches wirkstoffhaltige Pflaster enthält. Das erfindungsgemäße System zeichnet sich durch eine verbesserte Handhabung der wirkstoffhaltigen Pflaster aus. Darüber hinaus ermöglicht es eine Kontrolle, ob ein bestimmtes wirkstoffhaltiges Pflaster bereits angewendet wurde oder nicht.

Zur bestimmungsgemäßen Anwendung herkömmlicher, wirkstoffhaltiger Pflaster müssen typischerweise wenigstens die folgenden Schritte durchgeführt werden: Entnahme des Pflasters aus einer Verpackung (z.B. aus einem Siegelbeutel), Entfernung der die klebefähige Schicht des Pflasters bedeckenden Schutzfolie (*release liner*), Aufkleben des Pflasters auf der Haut und Entsorgung des Verpackungsmaterials (entleerte Verpackung und entfernte Schutzfolie).

Es besteht ein Bedarf, diesen Ablauf im Materialeinsatz zu reduzieren.

DE-A 100 56 234 offenbart eine Primärverpackung für medizinische Pflaster, welche eine flächenförmige Packstoffbahn, die zumindest in einem Teilbereich selbstklebend ausgerüstet ist, und eine haubenförmige, weitgehend verformungsstabile Abdeckung umfasst. Die Packstoffbahn und die Abdeckung sind in der Weise miteinander verbunden, dass ein für die Verpackung eines Pflasters geeigneter, allseitig umschlossener Innenraum gebildet wird, wobei der klebende Bereich der Packstoffbahn zum Innenraum hin gewandt ist und der Befestigung des zu verpackenden Pflasters über die Rückseite seiner Rückschicht dient. So ist es möglich, auf eine die Klebefläche bedeckende Schicht zu verzichten. Stattdessen ist es jedoch erforderlich, die Packstoffbahn zumindest in einem Teilbereich selbstklebend auszurüsten, was mit zusätzlichen Herstellungsschritten verbunden ist.

Darüber hinaus besteht ein Bedarf, die Handhabung wirkstoffhaltiger Pflaster zu vereinfachen. So haben ältere oder behinderte Menschen, aber auch Patienten mit bestimmten Erkrankungen, insbesondere mit neurodegenerativen Erkrankungen wie z.B. Morbus Alzheimer, mitunter Probleme, wirkstoffhaltige Pflaster selbständig anzuwenden und sich auf diese Weise die in den Pflastern enthaltenen Wirkstoffe ohne fremde Hilfe zu verabreichen.

Im Stand der Technik sind wirkstoffhattige Pflaster bekannt, welche einzeln in Schutzhüllen verpackt sind. Diese Schutzhüllen müssen unmittelbar vor der Anwendung der wirkstoffhaltigen Pflaster z.B. durch Aufschneiden, Aufreißen oder Ziehen an einem Aufreißstreifen bzw. einer Perforation geöffnet werden. Hinsichtlich der Handhabbarkeit ist diese Art der Verpackung jedoch mit Nachteilen behaftet. Insbesondere ältere oder behinderte Menschen haben Probleme, die Schutzhüllen zu öffnen. Im Falle des Aufschneidens mit Hilfe scharfer Gegenstände (Messer, Scheren, etc.) werden die wirkstoffhaltigen Pflaster vielfach beschädigt oder sogar unbrauchbar gemacht. Zudem ist diese Art der Verpackung vergleichsweise aufwändig und kostenintensiv. Weitere Nachteile sind eine erschwerte Entnahme des Pflasters aus der Hülle sowie ein Unbrauchbarwerden der Pflaster infolge einer Undichtigkeit der Schutzhülle. Ein weiterer Nachteil dieser Systeme ist, dass sie keinerlei Informationen bezüglich der Anwendungshäufigkeiten enthalten. Ferner hat Betreuungspersonal praktisch keinen Einblick, wie häufig die wirkstoffhaltigen Pflaster vom Patienten gewechselt werden.

Besondere Probleme können sich ergeben, wenn der Wirkstoff in den Pflastern in unterschiedlicher Dosierung vorliegt und es infolge eines streng einzuhaltenden Dosierschemas erforderlich ist, ein bestimmtes Pflaster auszuwählen und zum richtigen Zeitpunkt anzuwenden.

Es sind verschiedene Wirkstoffe bekannt, deren zu verabreichende tägliche Dosis nicht konstant ist, sondern im Laufe der Behandlung verändert werden muss. Dies kann aus verschiedenen Gründen erforderlich sein.

So ist es einerseits bekannt, dass bestimmte Wirkstoffe, insbesondere bestimmte Arzneistoffe, Nebenwirkungen zeigen, wenn direkt zu Beginn der Therapie die gesamte, für die Behandlung der jeweiligen Erkrankung erforderliche therapeutische Dosis verabreicht wird. In diesen Fällen können die Nebenwirkungen jedoch häufig reduziert werden, wenn zunächst nur eine Teildosis dieser therapeutischen Dosis verabreicht wird, und dann Schritt für Schritt die tägliche Dosis erhöht wird, bis schließlich die therapeutische Dosis erreicht ist (Titration). Man spricht in diesem Zusammenhang mitunter auch von einer "einschleichenden Behandlung", da in einer ersten Anpassungsphase der Organismus an den Wirkstoff gewöhnt wird, ohne dass dabei bereits die volle tägliche therapeutische Dosis erreicht wird. Diese Gewöhnung ist insbesondere bei solchen Wirkstoffen von Vorteil, bei denen der Bereich zwischen der therapeutischen Dosis und der maximal möglichen Dosis vergleichsweise schmal ist.

Ein Beispiel für einen Wirkstoff, bei dem zur Unterdrückung von Nebenwirkungen zu Beginn der Behandlung eine Steigerung der täglichen Dosis von Vorteil sein kann, ist Memantin, welches zur Behandlung von schwerer bis mittelschwerer Alzheimer-Erkrankung verwendet wird.

Andererseits sind auch Wirkstoffe bekannt, deren Dosis nach Ablauf eines gewissen Behandlungszeitraums erhöht werden muss, da ein Gewöhnungseffekt des Körpers eingetreten ist oder die Erkrankung zwischenzeitlich weiter fortgeschritten ist und daher höhere Dosen des Wirkstoffs erforderlich macht. Ein Beispiel für einen Wirkstoff, bei dem zur Aufrechterhaltung der therapeutischen Wirksamkeit eine Steigerung der täglichen Dosis von Vorteil sein kann, ist L-Dopa, welches zur Behandlung der Parkinson-Erkrankung verwendet wird.

Ein weiteres Beispiel für ein Dosierschema, bei dem die tägliche Dosis des zu verabreichenden Wirkstoffs nicht konstant ist, sind Kontrazeptiva, bei denen üblicherweise während einigen Tagen des Menstruationszyklus' die Verabreichung des Östrogens und/oder Gestagens unterbrochen wird, um dadurch die Entzugsblutung auszulösen. Auch während der Tage des Menstruationszyklus', an denen die Verabreichung von Östrogen und Gestagen erfolgt, kann deren jeweilige therapeutische Dosis variieren (mehrphasige Schemata).

Patienten mit eingeschränkten kognitiven Fähigkeiten sind jedoch häufig nur bedingt in der Lage, aus einer Verpackung, welche verschiedene wirkstoffhaltige Pflaster mit unterschiedlicher Dosierung enthält, das richtige Pflaster auszuwählen und anzuwenden. Probleme können sich auch bei der Handhabung des Pflasters ergeben. Solche Patienten benötigen mitunter die Unterstützung durch Pflegepersonal, welches in Altenheimen, Krankenhäusern oder auch zu Hause die korrekte Medikation überwacht. Da jedoch der Einsatz von Pflegepersonal mit Kosten verbunden ist, welche die sozialen Sicherungssysteme in erheblichem Maße finanziell belasten können, besteht ein grundsätzlicher Bedarf, den Einsatz von Pflegepersonal auf ein möglichst geringes Maß zu reduzieren. Dies kann in erster Linie durch die Mitwirkung des Patienten erreicht werden.

Es besteht somit ein Bedarf, die Handhabung von wirkstoffhaltigen Pflastern zu vereinfachen, um dadurch den Einsatz von Pflegepersonal reduzieren zu können.

DE-A 1-03 07 583 offenbart eine Primärverpackung, welche zur Applikation von wirkstoffhaltigen Systemen die Verwendung von Hilfsmitteln wie Handschuhen, Pinzetten oder dergleichen überflüssig macht, da der das Produkt auf die Applikationsstelle drückende Finger des Anwenders mit dem verpackten System nicht in Kontakt kommt.

WO 95/00122 offenbart ein Weiteres System umfassend mehrere Wirkstoffhaltige Pflaster.

Der Erfindung liegt die Aufgabe zugrunde, ein System bereitzustellen, welches wirkstoffhaltige Pflaster enthält und Vorteile gegenüber dem Stand der Technik aufweist. Die Anwendung der wirkstoffhaltigen Pflaster sollte vereinfacht sein, so dass z.B. auch Personen mit eingeschränkten kognitiven Fähigkeiten in die Lage versetzt werden, sich den in den Pflastern enthaltenen Wirkstoff zuverlässig selbst zu verabreichen. Dabei sollte auch die Einhaltung komplizierter Dosierschemata möglich sein. Darüber hinaus sollte das System dem Pflegepersonal eine möglichst zuverlässige Kontrolle ermöglichen, ob sich ein Patient die für den jeweiligen Zeitraum vorgesehene Wirkstoffdosis tatsächlich verabreicht hat oder nicht.

Diese Aufgabe wird durch den Gegenstand der Patentansprüche gelöst.

Es wurde gefunden, dass die Anwendung wirkstoffhaltiger Pflaster verbessert werden kann, wenn mehrere der wirkstoffhaltigen Pflaster über ihre klebefähige Schicht an einer gemeinsamen Schutzfolie haften. Auf diese Weise ist es auf einen Blick möglich, die Gesamtheit der in dem System ursprünglich enthaltenen wirkstoffhaltigen Pflaster zu überwachen. Enthält das erfindungsgemäße System beispielsweise 7 wirkstoffhaltige Pflaster, welche zur einmal täglichen Anwendung konfektioniert sind, so wird am ersten Tag der Verabreichung ein Pflaster von der gemeinsamen Schutzfolie abgelöst und auf eine geeignete Hautpartie des Patienten aufgebracht. Dadurch entsteht auf der gemeinsamen Schutzfolie an der Position, an welcher zuvor das Pflaster haftete, eine Leerstelle. Am dritten Tag der Verabreichung sollten sich demnach auf der Schutzfolie drei Leerstellen und noch vier wirkstoffhaltige Pflaster befinden, was durch bloße Sichtkontrolle schnell überprüft werden kann, beispielsweise durch Pflegepersonal, welches den Patienten nur einmal täglich oder nur in anderen regelmäßigen Abständen zu Hause aufsucht.

Gegenüber herkömmlichen Systemen, in denen die einzelnen wirkstoffhaltigen Pflaster lose vorliegen, hat das erfindungsgemäße System u.a. den Vorteil, dass die Kontrolle der Anwendung des Pflasters zuverlässiger ist. So kann einerseits die Kontrolle auf einen Blick erfolgen, ohne dass ein Zählen loser, in einer gemeinsamen Verpackung befindlicher Einheiten erforderlich wäre.

Darüber hinaus besteht bei herkömmlichen Systemen andererseits die Möglichkeit, dass der Patient einzelne der wirkstoffhaltigen Pflaster entnimmt und an einer anderen Stelle der Wohnung ablegt, ohne dass es zu der bestimmungsgemäßen Anwendung kommt. Bei den erfindungsgemäßen Systemen ist diese Möglichkeit eingeschränkt, da durch die Entnahme eines wirkstoffhaltigen Pflasters dessen klebefähige Schicht freigelegt wird. Bei unachtsamer bzw. unbewusster Handhabung bleibt es an den Händen haften, wodurch das wirkstoffhaltige Pflaster in das Bewusstsein des Patienten gerückt wird. Ein Ablegen an einer anderen Stelle der Wohnung wird auf diese Weise unwahrscheinlicher. Ist aber die Wahrscheinlichkeit vermindert, dass einzelne Pflaster einer nicht-bestimmungsgemäßen Anwendung zugeführt wurden, so steigt damit die Zuverlässigkeit der bloßen Sichtkontrolle.

Die Erfindung betrifft ein System umfassend mehrere, vorzugsweise 2, 3, 4, 5, 6, 7, 8, 9, 10, 11, 12, 13, 14 oder mehr wirkstoffhaltige Pflaster und eine gemeinsame Schutzfolie, wobei die Pflaster jeweils eine klebefähige Schicht aufweisen, über diese klebefähige Schicht an der gemeinsamen Schutzfolie haften und davon ablösbar sind, wobei jedes Pflaster jeweils von einer Abdeckfolie bedeckt ist, deren Fläche größer als die Fläche des Pflasters ist, wobei die Abdeckfolie in den Randbereichen ihrer dem Pflaster zugewandten Oberflächenseite ablösbar mit der gemeinsamen Schutzfolie verbunden ist, so dass die Abdeckfolie zusammen mit der gemeinsamen Schutzfolie einen geschlossenen Hohlraum bildet, in dem das Pflaster angeordnet ist.

Bei dem erfindungsgemäßen System ist die Abdeckfolie in den Randbereichen ihrer dem Pflaster zugewandten Oberflächenseite ablösbar mit der gemeinsamen Schutzfolie verbunden. Einem Fachmann ist bekannt, wie die Ablösbarkeit der Abdeckfolie von der gemeinsamen Schutzfolie verwirklicht werden kann.

Möglichkeiten zur Realisierung von Siegel- oder Klebstoffnähten (vgl. (iii)) sind dem Fachmann bekannt. Beispielsweise können die Abdeckfolie und die gemeinsame Schutzfolie jeweils als Mehrschichtfolien ausgebildet sein, wobei jeweils wenigstens eine der beiden äußeren Oberflächenschichten dieser Mehrschichtfolien eine Siegelschicht ist, d.h. auf einem heißsiegelbaren (Co-)Polymerisat basiert. Bevorzugt basiert die Siegelschicht auf wenigstens einem Polymer ausgewählt aus der Gruppe bestehend aus Polyolefinen, Olefin-Copolymerisaten und deren Mischung, insbesondere bevorzugt auf Polyethylen (z.B. HDPE, LDPE, LLDPE), Polypropylen, deren Copolymerisaten Und/oder Mischungen. Die Siegeltemperaturen liegen vorzugsweise im Bereich von 100°C bis 200°C. Die Schmelztemperatur der Siegelschicht beträgt vorzugsweise 90 bis 164°C, besonders bevorzugt 95°C bis 130°C. Die Siegelschicht kann mit üblichen Hilfsstoffen wie Antistatika, Gleitmitteln, Slipmitteln, Antiblockmitteln, Antifogmitteln und/oder Abstandshaltern ausgerüstet werden.

In einer bevorzugten Ausführungsform wird die Siegel- oder Klebstoffnaht durch einen Peel-Verschluss verwirklicht.

Die Erfindung betrifft ein System umfassend mehrere, vorzugsweise 2, 3, 4, 5, 6, 7, 8, 9, 10, 11, 12, 13, 14 oder mehr wirkstoffhaltige Pflaster und eine gemeinsame Schutzfolie, wobei die Pflaster jeweils eine klebefähige Schicht aufweisen, über diese klebefähige Schicht an der gemeinsamen Schutzfolie haften und davon ablösbar sind, wobei jedes Pflaster jeweils von einer Abdeckfolie bedeckt ist, deren Fläche größer als die Fläche des Pflasters ist und die wenigstens in den Randbereichen ihrer dem Pflaster zugewandten Oberflächenseite selbstklebend ist, wobei die Abdeckfolie über ihre selbstklebenden Randbereiche derart auf der gemeinsamen Schutzfolie haftet, dass sie zusammen mit dieser einen geschlossenen Hohlraum bildet, in dem das Pflaster angeordnet ist.

Bei dem erfindungsgemäßen System haften mehrere wirkstoffhaltige Pflaster an einer gemeinsamen Schutzfolie. Die gemeinsame Schutzfolie bedeckt dabei bei jedem wirkstoffhaltigen Pflaster bevorzugt zumindest den Wirkstoff abgebenden Bereich, vorzugsweise die gesamte klebefähige Schicht. Vor der Applikation des wirkstoffhaltigen Pflasters auf der Haut ist es von der gemeinsamen Schutzfolie leicht abziehbar.

Die gemeinsame Schutzfolie ist bevorzugt auf zumindest der Oberfläche, welche der klebefähigen Schicht zugewandt ist, silikonisiert und/oder fluoridiert. Die gemeinsame Schutzfolie kann ein Polymer enthalten ausgewählt aus der Gruppe bestehend aus Polyethylen, Polyester, Polyethylenterephthalat, Polypropylen, Polysiloxan, Polyvinylchlorid und Polyurethan. Gegebenenfalls kann sie behandelte Papierfasern oder Zellophan, enthalten und/oder gegebenenfalls eine Silikon-, Fluorsilikon- oder Fluorcarbonbeschichtung aufweisen. Bevorzugt basiert die gemeinsame Schutzfolie auf einem Polyester, beispielsweise auf Hostaphan^{®} RNT. Bevorzugt weist die gemeinsame Schutzfolie eine Dicke von 10 bis 1.000 µm, bevorzugter von 15 bis 500 µm, noch bevorzugter 20 bis 250 µm und insbesondere 25 bis 100 µm auf.

Vor der Anwendung des wirkstoffhaltigen Pflasters wird dieses von der gemeinsamen Schutzfolie abgelöst. Dadurch wird die Oberfläche der klebefähigen Schicht freigelegt. Bevorzugt grenzt die gemeinsame Schutzfolie unmittelbar an die klebefähige Schicht jedes wirkstoffhaltigen Pflasters an.

Die gemeinsame Schutzfolie kann je nach Anzahl der daran anhaftenden wirkstoffhaltigen Pflaster eine gewisse Größe erreichen. Bevorzugt ist sie mit Löchern, Schlaufen oder anderen Befestigungsmitteln ausgerüstet, welche die Anbringung der Schutzfolie an einer disponierten Stelle in der Wohnung oder des Zimmers des Patienten ermöglichen.

Erfindungsgemäß ist jedes wirkstoffhaltige Pflaster jeweils mit einer Abdeckfolie bedeckt. Die Fläche der Abdeckfolie ist größer als die Fläche des wirkstoffhaltigen Pflasters und weist wenigstens in ihren Randbereichen einen druckempfindlichen Klebstoff auf. Über diesen Randbereich haftet die Abdeckfolie auf der gemeinsamen Schutzfolie des erfindungsgemäßen Systems. Dabei weist der gesamte Randbereich der Abdeckfolie einen druckempfindlichen Klebstoff auf, so dass nach dem Anhaften der Abdeckfolie an der gemeinsamen Schutzfolie ein geschlossener Hohlraum aus Abdeck- und Schutzfolie gebildet wird, in dem ein wirkstoffhaltiges Pflaster angeordnet ist . Die Abdeckfolie kann mit einer Lasche oder einer anderen Vorrichtung ausgerüstet sein, die geeignet ist, das Abziehen der Abdeckfolie von der gemeinsamen Schutzfolie zu vereinfachen. Ist die Abdeckfolie transparent, so ist die Lasche vorzugsweise eingefärbt, damit sie besser erkannt werden kann.

Vorzugsweise ist die Abdeckfolie wenigstens in einem Bereich, vorzugsweise über seine gesamte Fläche, transparent. Vorzugsweise ist die Fläche des transparenten Bereichs mindestens so groß wie die Fläche des Pflasters, vorzugsweise größer. Bevorzugt kann die vollständige Fläche des Pflasters von außen durch den transparenten Bereich hindurch mit bloßem Auge betrachtet werden.

Durch die transparente Ausgestaltung der Abdeckfolie wird verhindert, dass das Pflaster beim Öffnen des aus Abdeckfolie und Schutzfolie gebildeten Hohlraums, beispielsweise mit Hilfe einer Schere, versehentlich beschädigt wird. So ermöglicht es der transparente Bereich, die Schere sicher am Pflaster vorbeizuführen.

Darüber hinaus ermöglicht die transparente Ausgestaltung der Abdeckfolie, Informationen nach außen sichtbar zu machen. Diese Informationen können als Kennzeichnungen beispielsweise auf das Pflaster oder auf die Oberfläche der Außenwand, welche dem Innenraum des aus Abdeckfolie und Schutzfolie gebildeten Hohlraums zugewandt ist, aufgedruckt sein. Durch die Abdeckfolie werden diese Kennzeichnungen vor äußeren mechanischen Einflüssen geschützt. Zur Kennzeichnung kommen insbesondere Symbole, Zahlen und/oder Buchstaben in Frage. Als Kennzeichnungen eignen sich insbesondere Produktbezeichnungen (z.B. Markennamen), Tageszeiten (z.B. morgens, mittags, abends, nachts), Uhrzeiten, Namen von Wochentagen oder deren Abkürzungen, Namen von Monaten oder deren Abkürzungen, Wirkstoffnamen (z.B. Buprenorphin) und Wirkstoffdosierungen (z.B. 250 mg, 500 mg) bzw. Freisetzungsraten (z.B. 35 µg/h, 70 µg/h).

Der Begriff "transparent" im Sinne der Erfindung bedeutet, dass ein Pflaster durch die Abdeckfolie hindurch mit bloßem Auge betrachtet werden kann. Die Transparenz wird bevorzugt mit Hilfe von Densitometem quantifiziert. Derartige Methoden sind dem Fachmann geläufig. Bevorzugt kann als Maß für die Transparenz die Trübung als optischer Wert gemessen werden. Die Messung der Trübung erfolgt bevorzugt nach der ASTM-Prüfnorm D 1003-61 m, Procedure A. Die erfindungsgemäße Abdeckfolie weist bevorzugt wenigstens in einem Teilbereich eine Trübung von weniger als 30%, bevorzugter weniger als 25%, noch bevorzugter weniger als 20%, am bevorzugtesten weniger als 15% und insbesondere weniger als 12% auf. Geeignete Materialien zur Herstellung transparenter Abdeckfolien sind dem Fachmann bekannt. In diesem Zusammenhang kann beispielsweise vollumfänglich verwiesen werden auf L. Massey, Permeability Properties of Plastics and Elastomers: A Guide to Packaging and Barrier Materials; Plastics Design Library; 2nd ed, 2003; H. Lockhart et al., Packaging Pharmaceutical and Healthcare Products, Springer; 1 ed, 2006; und D.A. Dean, Pharmaceutical Packaging Technology, Taylor & Francis; 1 ed, 2000.

Um den Schutz vor UV-Strahlung zu verbessern, kann der transparente Bereich eingefärbt sein. Um eine ausreichende Luftdichtigkeit zu erzielen, kann der transparente Bereich mehrschichtig ausgebildet sein und eine Barriereschicht umfassen, welche vorzugsweise auf (Co-)-Polyamid, Ethylen-Vinylalkohol-Copolymerisat (EVOH) oder Polyvinylidenchlorid basiert.

Die vorzugsweise transparente Abdeckfolie kann als zusätzlicher Schutz für die wirkstoffhaltigen Pflaster dienen, beispielsweise wenn die wirkstoffhaltigen Pflaster feuchtigkeitsempfindlich sind oder vor äußeren mechanischen Einwirkungen geschützt werden müssen.

Darüber hinaus gewährleistet die Abdeckfolie, dass die gemeinsame Schutzfolie auch dann noch problemlos aus einer Umverpackung herausgenommen werden kann, wenn es im Randbereich einzelner Pflaster zu einem "*cold flow*" gekommen ist. So verhindert die Abdeckfolie, dass der im Randbereich der Pflaster infolge des "*cold flow*" feigelegte Klebstoff an der Umverpackung haftet. Auch für Pflegepersonal bietet dies einen zusätzlichen Schutz, da beim Herausnehmen der gemeinsamen Schutzfolie aus der Umverpackung ein Berühren des ggf. wirkstoffhaltigen, freigelegten Klebstoffs mit den Fingern durch die Abdeckfolie verhindert wird.

Die vorzugsweise transparente Abdeckfolie ist vollflächig selbstklebend ausgerüstet. So haftet sie auch auf der Oberflächenseite des wirkstoffhaltigen Pflasters, welche der klebefähigen Schicht abgewandt ist. In diesem Fall kann die vorzugsweise transparente Abdeckfolie ggf. als Applikationshilfe verwendet werden. Somit liegen folgende Wechselwirkungen über selbstklebende Bereiche vor:
(i) Haftung der klebefähigen Schicht des Pflasters auf der gemeinsamen Schutzfolie;
(ii) Haftung der selbstklebenden Abdeckfolie auf der Rückseite des Pflasters, welche der klebefähigen Schicht des Pflasters abgewandt ist, und
(iii) Haftung der selbstklebenden Abdeckfolie auf der gemeinsamen Schutzfolie im Randbereich der Abdeckfolie.

Vorzugsweise ist die Klebkraft der Haftung (i) vergleichbar mit der Klebkraft der Haftung (iii) und jeweils kleiner als die Klebkraft der Haftung (ii). Auf diese Weise wird gewährleistet, dass das Pflaster zusammen mit der Abdeckfolie von der gemeinsamen Schutzfolie abgelöst werden kann, ohne dass sich (zunächst) auch die Abdeckfolie von der Rückseite des Pflasters ablöst. Die vorzugsweise transparente Abdeckfolie kann nun als Applikationshilfe verwendet und nach der bestimmungsgemäßen Anwendung des Pflasters auf der Haut des Patienten abgelöst werden. Dazu ist vorzugsweise die Klebkraft der Haftung der klebefähigen Schicht des Pflasters auf der Haut des Patienten größer als die Klebkraft der Haftung (ii).

Es wurde überraschend gefunden, dass mit Hilfe einer transparenten Applikationshilfe die Anwendung des Pflasters vereinfacht wird. So kann die Stelle der Haut, auf welcher das Pflaster angebracht werden soll, durch die transparente Applikationshilfe hindurch anvisiert werden, was die Zuverlässigkeit der Positionierung deutlich erhöht.

In einer alternativen Ausführungsform weist die vorzugsweise transparente Abdeckfolie auf ihrer dem Pflaster zugewandten Oberfläche einen druckempfindlichen Klebstoff auf, jedoch nicht in ihren Randbereichen. Die vorzugsweise transparente Abdeckfolie haftet auf der Rückseite des Pflasters, welche der klebefähigen Schicht des Pflasters abgewandt ist. Die Schutzfolie weist ihrerseits in den Bereichen, welche den Randbereichen der Abdeckfolie gegenüberliegen, einen druckempfindlichen Klebstoff auf. Die gemeinsame Schutzfolie haftet über diese Randbereiche derart auf der Abdeckfolie, dass sie zusammen mit dieser einen geschlossenen Hohlraum bildet, in dem das Pflaster angeordnet ist. Der druckempfindliche Klebstoff auf der Schutzfolie umgibt das Pflaster wie einen Rahmen. Bei dieser Ausführungsform liegen somit folgende Wechselwirkungen über selbstklebende Bereiche vor:
(i) Haftung der klebefähigen Schicht des Pflasters auf der gemeinsamen Schutzfolie;
(ii) Haftung der selbstklebenden Abdeckfolie auf der Rückseite des Pflasters, welche der klebefähigen Schicht des Pflasters abgewandt ist; und
(iii) Haftung der gemeinsamen Schutzfolie auf der Abdeckfolie über die selbstklebenden Bereiche der Schutzfolie, welche den Randbereichen der Abdeckfolie gegenüberliegen.

Vorzugsweise ist auch bei dieser Ausführungsform die Klebkraft der Haftung (i) vergleichbar mit der Klebkraft der Haftung (iii) und jeweils kleiner als die Klebkraft der Haftung (ii). Auf diese Weise wird gewährleistet, dass das Pflaster zusammen mit der Abdeckfolie von der gemeinsamen Schutzfolie abgelöst werden kann, ohne dass sich (zunächst) auch die Abdeckfolie von der Rückseite des Pflasters ablöst. Die vorzugsweise transparente Abdeckfolie kann nun als Applikationshilfe verwendet und nach der bestimmungsgemäßen Anwendung des Pflasters auf der Haut des Patienten abgelöst werden. Dazu ist vorzugsweise die Klebkraft der Haftung der klebefähigen Schicht des Pflasters auf der Haut des Patienten größer als die Klebkraft der Haftung (ii). Diese Ausführungsform hat den Vorteil, dass nach Ablösen von Pflaster und daran haftender Abdeckfolie von der gemeinsamen Schutzfolie der freigelegte Randbereich der Abdeckfolie seinerseits nicht selbstklebend ist. Die Anwendung der vorzugsweise transparenten Abdeckfolie ,als Applikationshilfe wird dadurch vereinfacht, da sie im Randbereich weder an den Fingern, noch auf der Haut des Patienten in der Umgebung des Pflasters haftet.

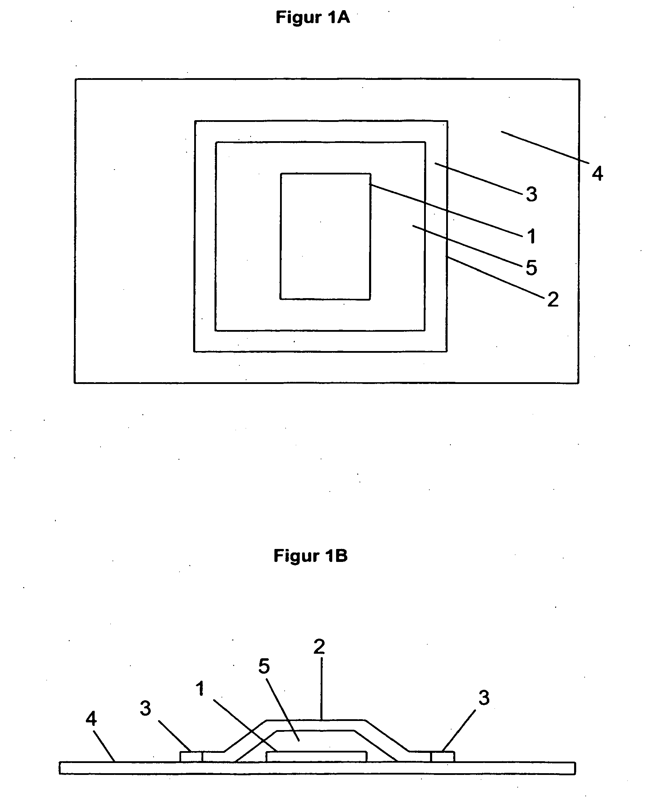

Figur 1 zeigt einen schematisierten Ausschnitt nicht erfindungsgemäßer Ausführungsformen eines Systems, Figur 1A in der Draufsicht, Figur 1B in der Seitenansicht. Mindestens ein Pflaster (1) ist von einer Abdeckfolie (2) bedeckt, deren Fläche größer als die Fläche des Pflasters (1) ist, wobei
(i) die Abdeckfolie (2) wenigstens in den Randbereichen (3) ihrer dem Pflaster (1) zugewandten Oberflächenseite selbstklebend ist und die Abdeckfolie (2) über ihre selbstklebenden Randbereiche (3) auf der gemeinsamen Schutzfolie (4) haftet;
   oder
(ii) die gemeinsame Schutzfolie (4) in den Bereichen, welche den Randbereichen (3) der Abdeckfolie gegenüberliegen, selbstklebend ist, und die gemeinsame Schutzfolie (4) über diese selbstklebenden Bereiche auf der Abdeckfolie (2) haftet;
   oder
(iii) die Abdeckfolie (2) in den Randbereichen (3) ihrer dem Pflaster (1) zugewandten Oberflächenseite über eine Siegel- oder Klebstoffnaht mit der gemeinsamen Schutzfolie (4) verbunden ist;
   so dass die Abdeckfolie (2) zusammen mit der gemeinsamen Schutzfolie (4) einen geschlossenen Hohlraum (5) bildet, in dem das Pflaster (1) angeordnet ist.

In einer bevorzugten Ausführungsform des erfindungsgemäßen Systems weisen die einzelnen wirkstoffhaltigen Pflaster gleiche oder verschiedene Markierungen auf. Sind die wirkstoffhaltigen Pflaster mit Abdeckfolien abgedeckt, so können auch die Abdeckfolien gleiche oder verschiedene Markierungen aufweisen.

Bevorzugt können alle wirkstoffhaltigen Pflaster des Systems in Gruppen eingeteilt werden, wobei sich wirkstoffhaltige Pflaster derselben Gruppe durch gleiche Markierungen und wirkstoffhaltige Pflaster verschiedener Gruppen durch verschiedene Markierungen auszeichnen. Bevorzugt umfasst jede Gruppe wenigstens zwei, bevorzugter wenigstens drei wirkstoffhaltige Pflaster.

Dabei kann es bevorzugt sein, dass wirkstoffhaltige Pflaster derselben Gruppe identischen Aufbau, insbesondere die gleiche Größe, die gleiche Dosierung des gleichen Wirkstoffs und die gleiche Freisetzungscharakteristik aufweisen, wohingegen wirkstoffhaltige Pflaster unterschiedlicher Gruppen sich vorzugsweise in wenigstens einer dieser Eigenschaften unterscheiden. Diese Ausführungsform ist insbesondere dann von Vorteil, wenn mit Hilfe der wirkstoffhaltigen Pflaster verschiedener Gruppen ein bestimmtes Dosierschema eingehalten oder ermöglicht werden soll.

Es kann aber auch bevorzugt sein, dass alle wirkstoffhaltigen Pflaster identischen Aufbau haben, unabhängig davon, zu welcher Gruppe sie gehören. So kann beispielsweise das erfindungsgemäße System 28 wirkstoffhaltige Pflaster enthalten, von denen jeweils 4 mit Abkürzungen für die 7 Wochentage, z.B. mit Mo, Di, Mi, Do, Fr, Sa bzw. So bedruckt sind. In diesem Fall kann die Markierung insbesondere dem Patienten bzw. dem Pflegepersonal eine Hilfe bei der Überprüfung sein, ob ein für einen bestimmten Zeitpunkt vorgesehene Applikation eines wirkstoffhaltigen Pflasters bereits stattgefunden hat oder noch nicht. Erfolgt die Anwendung eines einzelnen wirkstoffhaltigen Pflasters über längere Zeiträume, beispielsweise über mehrere Tage, so kann die Markierung auch bei der Kontrolle hilfreich sein, ob ein bestimmtes, momentan auf der Haut des Patienten appliziertes wirkstoffhaltiges Pflaster gewechselt werden muss oder noch nicht.

Bevorzugt sind die Markierungen Einfärbungen und/oder Bedruckungen, insbesondere Symbole, Schriftzeichen, Buchstaben oder Zahlen, welche vorzugsweise für Uhrzeiten (z.B. 7 Uhr, morgens, mittags, nachmittags, abends, nachts), Wochentage oder deren Abkürzungen stehen.

Die Markierungen, mit denen die Pflaster und/oder die gemeinsame Schutzfolie bedruckt sein kann, können dem Pflegepersonal darüber hinaus dabei helfen, gesetzlichen Bestimmungen Rechnung zu tragen, welche im Zusammenhang mit bestimmten Wirkstoffen eingehalten werden müssen. So kann das erfindungsgemäße System als eine Art Vorratspackung wirkstoffhaltiger Pflaster in Krankenhäusern verwendet werden. Fällt der in den wirkstoffhaltigen Pflastern enthaltene Wirkstoff unter das Betäubungsmittelgesetz, wie z.B. Buprenorphin, so sind durch das Pflegepersonal bestimmte Auflagen einzuhalten, insbesondere ist die Anwendung der wirkstoffhaltigen Pflaster zu dokumentieren. Die Dokumentation des Verbleibs einzelner Pflaster kann z.B. durch Markierungen, z.B. Nummerierungen, auf der gemeinsamen Schutzfolie erleichtert werden.

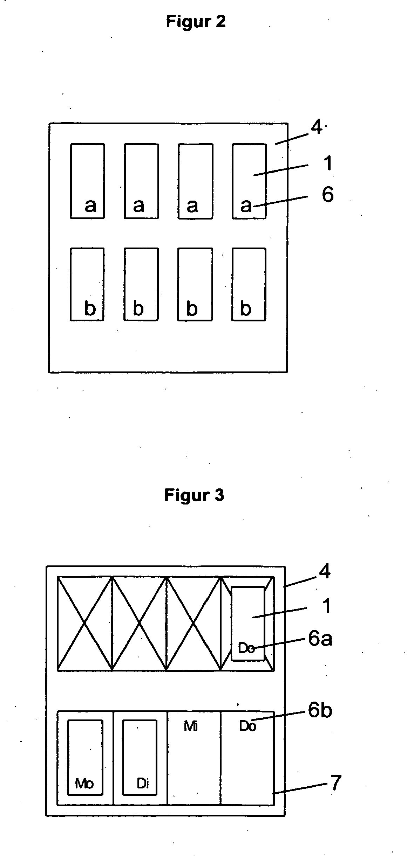

Figur 2 zeigt eine bevorzugte Ausführungsform des erfindungsgemäßen Systems. Acht wirkstoffhaltige Pflaster (1) haften an einer gemeinsamen Schutzfolie (4) und sind in zwei Reihen zu je vier Pflastern (1) angeordnet. Jedes wirkstoffhaltige Pflaster (1) ist mit einer Markierung (6) versehen, wobei die Markierung bei den vier wirkstoffhaltigen Pflastern (1) der oberen Reihe der Buchstabe "a", bei den vier wirkstoffhaltigen Pflastern (1) der unteren Reihe der Buchstabe "b" ist. Anhand der Markierung (6) lassen sich die acht wirkstoffhaltigen Pflaster (1) des Systems in zwei Gruppen einteilen, wobei die eine Gruppe von den wirkstoffhaltigen Pflastern (1) der oberen Reihe mit der Markierung "a" und die andere Gruppe von den wirkstoffhaltigen Pflastern (1) der unteren Reihe mit der Markierung "b" gebildet wird.

In einer bevorzugten Ausführungsform des erfindungsgemäßen Systems weist die gemeinsame Schutzfolie für jedes Pflaster bzw. für jede Abdeckfolie eine der Markierung des jeweiligen Pflasters bzw. der jeweiligen Abdeckfolie entsprechende oder davon verschiedene Markierung auf. Auf diese Weise wird die Blickkontrolle vereinfacht, d.h. es wird ermöglicht, dass mit einem Blick auf die gemeinsame Schutzfolie ersichtlich ist, welche Markierung die noch an der gemeinsamen Schutzfolie haftenden wirkstoffhaltigen Pflaster aufweisen und welche Markierung die bereits von der gemeinsamen Schutzfolie abgelösten wirkstoffhaltigen Pflaster aufweisen.

Bevorzugt werden bei dieser Ausführungsform die Markierungen der gemeinsamen Schutzfolie für ein bestimmtes Pflaster erst dann nach außen sichtbar, wenn dieses Pflaster von der gemeinsamen Schutzfolie abgelöst worden ist.

In einer weiteren bevorzugten Ausführungsform weist das erfindungsgemäße System eine Vorrichtung auf, um die Pflaster nach deren bestimmungsgemäßer Anwendung aufzunehmen. Diese Vorrichtung kann beispielsweise als vorzugsweise transparenter Beutel ausgebildet sein. Diese Vorrichtung ermöglicht die Kontrolle, wie viele wirkstoffhaltige Pflaster bereits angewendet wurden.

Diese Ausführungsform verbessert die Zuverlässigkeit der bloßen Sichtkontrolle. Wird eine Diskrepanz festgestellt zwischen der Anzahl der ursprünglich im System vorhandenen wirkstoffhaltigen Pflaster und der Summe der noch nicht von der gemeinsamen Schutzfolie abgelösten wirkstoffhaltigen Pflaster, der momentan beim Patienten angewendeten wirkstoffhaltigen Pflaster und der in der Vorrichtung befindlichen wirkstoffhaltigen Pflaster, so erfolgte möglicherweise keine ordnungsgemäße Anwendung, da offenbar eines oder mehrere der wirkstoffhaltigen Pflaster nicht der bestimmungsgemäßen Anwendung zugeführt wurden. Besteht eine solche Diskrepanz hingegen nicht, so ist die Wahrscheinlichkeit vergleichsweise hoch, dass die Anwendung aller wirkstoffhaltigen Pflaster bisher ordnungsgemäß erfolgt ist.

Die Vorrichtung zur Aufnahme der wirkstoffhaltigen Pflaster nach deren bestimmungsgemäßer Anwendung kann auch ein Bestandteil der gemeinsamen Schutzfolie sein. Beispielsweise können die wirkstoffhaltigen Pflaster an einer gemeinsamen Schutzfolie haften, welche räumlich davon getrennt, beispielsweise auf ihrer Rückseite, markierte Positionen aufweist, auf denen die wirkstoffhaltigen Pflaster - nach ihrer bestimmungsgemäßen Anwendung wieder aufgeklebt werden können. Diese Positionen können ggf. ihrerseits selbstklebend ausgerüstet sein, um eine mögliche Schwächung der Klebkraft der klebefähigen Schicht der wirkstoffhaltigen Pflaster im Zuge ihrer bereits erfolgten Anwendung auf der Haut auszugleichen und ggf. eine erneute, irrtümliche Abnahme zu verhindern.

Figur 3 zeigt eine bevorzugte Ausführungsform des erfindungsgemäßen Systems, bei dem ursprünglich vier wirkstoffhaltige Pflaster (1) an einer gemeinsamen Schutzfolie (4) gehaftet haben. Die vier wirkstoffhaltigen Pflaster (1) sind mit Markierungen (6a) versehen, welche im vorliegenden Fall für die Wochentage Montag (Mo), Dienstag (Di), Mittwoch (Mi) und Donnerstag (Do) stehen. Im ursprünglichen Zustand waren alle vier wirkstoffhaltigen Pflaster (1) nebeneinander in einer Reihe parallel zu dem mit "Do" markierten Pflaster angeordnet. Die Vorrichtung zur Aufnahme der wirkstoffhaltigen Pflaster nach deren bestimmungsgemäßer Anwendung (7) ist auf dem unteren Abschnitt der gemeinsamen Schutzfolie (4) angeordnet und wies in ursprünglichen Zustand kein wirkstoffhaltiges Pflaster (1) auf. Im abgebildeten Zustand wurden jedoch die mit "Mo" und "Di" markierten wirkstoffhaltigen Pflaster (1) bereits bestimmungsgemäß angewendet und danach auf der Vorrichtung (7) befestigt. Die Vorrichtung (7) enthält Markierungen (6b), welche den Markierungen (6a) jeweils entsprechen. Das mit "Mi" markierte wirkstoffhaltige Pflaster (1) ist nicht abgebildet und befindet sich bestimmungsgemäß auf der Haut des Patienten. Auf diese Weise kann durch bloße Sichtkontrolle die Vollständigkeit des Systems überprüft werden.

Gemäß des erfindungsgemäßen Systems ist jedes wirkstoffhaltige Pflaster auf seiner Oberflächenseite, welche der klebefähigen Schicht abgewandt ist, jeweils mit einer selbstklebenden und ablösbaren, vorzugsweise transparenten Applikationshilfe versehen. Die vorzugsweise transparente Applikationshilfe kann mehrteilig sein, vorzugsweise ist sie zweiteilig. Vorzugsweise ist die Haftkraft der Applikationshilfe auf der Oberflächenseite des Pflasters, welche der klebefähigen Schicht abgewandt ist, größer als die Haftkraft der klebefähigen Schicht des Pflasters auf der gemeinsamen Schutzfolie. Auf diese Weise wird gewährleistet, dass bei Ausübung einer Zugkraft auf die vorzugsweise transparente Applikationshilfe diese mitsamt dem wirkstoffhaltigen Pflaster von der gemeinsamen Schutzfolie abgelöst wird, also nicht das wirkstoffhaltige Pflaster auf der gemeinsamen Schutzfolie zurückbleibt.

Die Funktionsweise der vorzugsweise transparenten Applikationshilfe kann darauf beruhen, dass sie aus einem Material gebildet ist, welches eine höhere Rigidität als das wirkstoffhaltige Pflaster aufweist. Auf diese Weise wird die Handhabung des Pflasters beim Ablösen von der gemeinsamen Schutzfolie und Aufbringen auf der Haut vereinfacht. Auch die Gefahr, dass der Patient bzw. eine Pflegeperson bei der Applikation des Pflasters versehentlich mit den Fingern die ggf. vorhandene, wirkstoffhaltige Schicht berührt, kann durch die Applikationshilfe vermindert werden.

Bestimmungsgemäß wird die vorzugsweise transparente Applikationshilfe erst dann vom wirkstoffhaltigen Pflaster abgelöst, wenn dieses auf der Haut des Patienten haftet. Dazu ist bevorzugt die Haftkraft der Applikationshilfe auf der Oberflächenseite des Pflasters, welche der klebefähigen Schicht abgewandt ist, kleiner als die Haftkraft der klebefähigen Schicht des Pflasters auf der menschlichen Haut.

Die Größe von wirkstoffhaltigen Pflastern wird mit zunehmendem wissenschaftlichen Fortschritt immer geringer, so dass es auch immer schwieriger wird, wirkstoffhaltige Pflaster mit einem wirkstofffreien Rand zu umgeben. Die Gefahr von Kontaminationen, beispielsweise bei Pflegepersonal, welches versehentlich die wirkstoffhaltige Fläche des Pflasters berührt, kann durch die Applikationshilfe wirksam vermindert werden.

Die Applikationshilfe ist vorzugsweise eine ggf. mehrschichtige, vorzugsweise transparente Polymerfolie, ein Papierlaminat oder eine Metallfolie.

Die Pflaster des erfindungsgemäßen Systems sind wirkstoffhaltig.

In einer nicht erfindungsgemäßen Ausführungsform sind jedoch nur einige Pflaster wirkstoffhaltig, andere Pflaster sind hingegen wirkstofffrei. Diese Ausführungsform eignet sich besonders zur Intervalltherapie, d.h. zu einer Therapie, bei der zwischen zwei aufeinander folgenden Dosierungsintervallen eines Wirkstoffs ein gewisser Zeitraum ohne Wirkstoffgabe verstreicht. Um die Einhaltung dieses zwischen zwei Behandlungsintervallen liegenden Zeitraums zu vereinfachen, erfolgt währenddessen die Verabreichung eines Placebos, hier die Anwendung eines wirkstofffreien Pflasters. Beispiele für Wirkstoffe, welche durch Intervalltherapie verabreicht werden, sind lokal wirkende Corticoide und systemisch wirkende Kontrazeptiva.

Zum Zwecke der Beschreibung umfasst der Begriff "wirkstoffhaltiges Pflaster" eine selbstklebend ausgerüstete Vorrichtung, welche als Wirkstoff wenigstens eine physiologisch wirksame Substanz enthält. Als Wirkstoffe kommen insbesondere Arzneistoffe (pharmazeutische Wirkstoffe), aber beispielsweise auch Nahrungsergänzungsmittel in Betracht.

Der Wirkstoff kann einerseits dazu befähigt sein, die Haut zu durchdringen und in die Blutbahn aufgenommen zu werden, so dass es sich bei dem erfindungsgemäßen wirkstoffhaltigen Pflaster in diesem Fall um ein transdermales therapeutisches System (TTS) handelt. In diesem Fall erfolgt die Wirkung des Wirkstoffs systemisch. Andererseits kann der Wirkstoff jedoch auch nicht dazu befähigt sein, die Haut zu durchdringen. In diesem Fall erfolgt die Wirkung topisch bzw. lokal.

In der Therapie haben sich wirkstoffhaltige Pflaster bewährt, da sie eine schmelzlose, bequeme und einfache Verabreichung eines Wirkstoffes an den Patienten über einen längeren Zeitraum ermöglichen.

Üblicherweise umfasst ein wirkstoffhaltiges Pflaster wenigstens
a) eine für den Wirkstoff undurchlässige Trägerschicht,
b) eine klebefähige Schicht, welche die Trägerschicht zumindest teilweise bedeckt, und
c) ggf. eine Schutzfolie, welche die klebefähige Schicht zumindest teilweise bedeckt und davon abziehbar ist; bei den wirkstoffhaltigen Pflastern des erfindungsgemäßen Systems teilen sich wenigstens zwei wirkstoffhaltige Pflaster eine gemeinsame Schutzfolie (siehe oben).

Hinsichtlich des Aufbaus und der Funktionsweise kann bei wirkstoffhaltigen Pflastern zwischen membrangesteuerten und matrixgesteuerten Pflastern unterschieden werden.

Ein Reservoir-Pflaster umfasst üblicherweise einen auf der Haut selbstklebenden, flachen Beutel, welcher die Wirkstoffe dispergiert enthält. Der Hautseite zugewandt ist der Beutel mit einer für den Wirkstoff durchlässigen Membran ausgerüstet, welche die Wirkstofffreigabe steuert. Klebefähige Schicht und wirkstoffhaltige Schicht (Reservoir) sind üblicherweise räumlich voneinander getrennte Untereinheiten eines Reservoir-Pflasters. Die klebefähige Schicht muss dann ihrerseits zur Aufnahme des Wirkstoffs befähigt sein, denn der Wirkstoff muss die klebefähige Schicht durchdringen, um von der wirkstoffhaltigen Schicht zur Haut zu gelangen. Derartige Pflaster weisen somit gewissermaßen zwei wirkstoffhaltige Schichten auf, von denen eine Schicht zusätzlich klebefähig ist, die andere jedoch nicht.

Bei einem Matrix-Pflaster ist der Wirkstoff in einer Matrix eingebettet, wobei er in flüssigem, halbfestem oder festem Zustand dispergiert oder in gelöster Form vorliegen kann. Weist die Matrix gleichzeitig klebefähige Eigenschaften auf, so ist die klebefähige Schicht gleichzeitig auch die wirkstoffhaltige Schicht. Es ist aber auch möglich, dass die klebefähige Schicht und die wirkstoffhaltige Schicht - wie auch bei einem Reservoir-Pflaster - voneinander getrennt sind, wobei auch in diesem Fall die klebefähige Schicht zur Aufnahme des Wirkstoffs geeignet sein muss. In diesem Fall kann die klebefähige Schicht flächendeckend oder partiell, z.B. ringförmig auf der wirkstoffhaltigen Schicht bzw. der Membran des Reservoirs aufgebracht sein kann.

Durch die Zusammensetzung und Strukturierung der Matrix und/oder der Membran kann die Freisetzung des Wirkstoffs reguliert werden. Hinsichtlich weiterer Einzelheiten kann beispielsweise auf T.K. Gosh, Transdermal and Topical Drug Delivery Systems Es Into Practice, CRC Press, 1997; R.O. Potts et al., Mechanisms of Transdermal Drug Delivery (Drugs and the Pharmaceutical Sciences), Marcel Dekker, 1997; und R. Gurny et al, Dermal and Transdermal Drug Delivery. New Insights and Perspectives, Wissenschaftliche VG., Stuttgart, 1998 verwiesen werden.

Damit ein Wirkstoff mit Hilfe eines wirkstoffhaltigen Pflasters transdermal verabreicht werden kann (bei dem wirkstoffhaltigen Pflaster handelt es sich dann um ein TTS), muss der Wirkstoff in ausreichender Menge durch die Epidermis der Haut, insbesondere auch das *Stratum Corneum* diffundieren, die einzelnen darunter liegenden Hautschichten passieren und vom Blutkreislauf aufgenommen werden. Ein wesentliches Problem stellt dabei häufig die nur geringe Durchlässigkeit der Haut für bestimmte Wirkstoffe dar. Je nach chemischer Struktur und Lipophilie des Wirkstoffs kann es daher erforderlich sein, die transkutane Permeation des Wirkstoffs aus dem TTS durch Zusatz geeigneter Hilfsstoffe zu verbessern.

Neben den gewünschten pharmakokinetischen Parametern muss ein wirkstoffhaltiges Pflaster eine ausreichende Hautverträglichkeit aufweisen. Die Haftung des wirkstoffhaltigen Pflasters auf der Haut wird üblicherweise mit Hilfe druckempfindlicher Klebstoffe (medizinischer Haftkleber) erreicht. Um die chemischen und/oder physikalischen Eigenschaften, z.B. die Kohärenz oder die Klebrigkeit medizinischer Haftkleber zu modifizieren und in Abhängigkeit von den jeweiligen Erfordernissen zu optimieren, werden üblicherweise bestimmte Hilfs- oder Zusatzstoffe zugesetzt, insbesondere Weichmacher oder Klebrigmacher (*tackifier*). Diese Hilfs- oder Zusatzstoffe sollten jedoch mit den übrigen verwendeten Materialien kompatibel sein. So sollten die genannten Hilfs- oder Zusatzstoffe die chemische Stabilität der enthaltenen Wirkstoffe oder deren Freisetzung nicht negativ beeinflussen. Häufig kann es auch vorteilhaft sein, wenn die Polymermatrix eines wirkstoffhaltigen Haftklebers überwiegend lipophile Eigenschaften aufweist, damit sie eine hohe Beladungskapazität für lipophile Wirkstoffe besitzt.

Die wirkstoffhaltigen Pflaster des erfindungsgemäßen Systems, vorzugsweise die klebefähigen Schichten der wirkstoffhaltigen Pflaster, können als Wirkstoff eine oder mehrere, vorzugsweise transdermal verabreichbare, physiologisch wirksame Substanzen enthalten, beispielsweise Arzneistoffe (pharmazeutische Wirkstoffe), aber auch Nahrungs- oder Nahrungsergänzungsmittel. Der Wirkstoff kann systemisch oder topisch wirksam sein. Vorzugsweise enthalten die wirkstoffhaltigen Pflaster wenigstens einen transdermal verabreichbaren Wirkstoff, welcher systemisch wirksam ist. Der Wirkstoff kann dabei in verschiedenen physikalischen Zuständen vorliegen, wie molekular verteilt, als Kristalle, in Form von Clustern oder eingekapselt in Liposomen.

Beispiele für vorzugsweise transdermal verabreichbare Nahrungs- oder Nahrungsergänzungsmittel sind Proteine, Saccharide, Lipide, Vitamine, Provitamine, Spurenelemente, gesättigte oder ungesättigte Fettsäuren und Antioxidanzien.

Bevorzugt enthalten die wirkstoffhaltigen Pflaster einen oder mehrere Wirkstoffe, vorzugsweise allerdings nur einen einzigen transdermal zu verabreichenden Wirkstoff. Der Wirkstoff ist vorzugsweise zumindest zum Teil in eine Matrix eingebettet.

Vorzugsweise ist der Wirkstoff ausgewählt aus der Gruppe bestehend aus Antiallergika; Antiarthritika; Antiasthmatika; Antibiotika und anderen antimikrobiellen Mitteln, wie Tetracyclinen, Oxytetracyclinen, Chlortetracyclinen, Sulfonamiden; Antidepressiva; Antidiabetika, wie Insulin; Antiepileptika; Antihistaminika; .Antihypertonika; Anti-Krampfmitteln, wie Atropin, Butylscopolamin-Bromid; Antimigränemitteln; Antipyretika; Antirheumatika; antiviralen Mitteln; Anxiolytika; Cardiaca; cardiovasculären Verbindungen, wie Nitroglycerin, cardialen Glycosiden; Coronardilatatoren; Corticosteroiden; entzündungshemmenden Mitteln; Enzymen; Fungiziden; Immunmodulatoren; Impfstoffen; Kontrazeptiva;, Lokalanästhetika; Mitteln zur Behandlung von Alkohol- und/oder Drogen- und/oder Medikamentenabhängigkeit; Mitteln zur Behandlung von Erkrankungen des zentralen Nervensystems; Mitteln zur Behandlung von neurodegenerativen Erkrankungen, insbesondere zur Behandlung von Morbus Parkinson .und/oder Morbus Alzheimer; Narkotika und Analgetika, wie Benzocain, Lidocain, Prilocain; nicht steriodalen Antirheumatika oder entzündungshemmenden Verbindungen, wie Indometacin, Diclofenac; Nicotin; Opioiden (einschließlich Opiaten); Psychopharmaka, wie 3-(2-Aminopropyl)indolacetat und 3-(2-Aminobutyl)indolacetat; Sedative wie Pentabarbiturat-Natrium, Phenobarbiturat, Secobarbiturat-Natrium, Codein, Carbromat; (Sexual-)Hormonen, wie Adrenocorticosteroiden, wie 6-a-Methylprednisolon, androgenen Steroiden, wie Methyltestosteroide, Fluoxymesteron, östrogenen Steroiden, wie Östrogen, 17-β-Estradiol, Ethinylestradiol, Progesteron, Norethindron, Thyroxin; Stimulantien; Tranquilizern, wie Reserpin, Chlorpromazin-Hydrochlorid; und Vitaminen.

In einer bevorzugten Ausführungsform des erfindungsgemäßen Systems enthalten die wirkstoffhaltigen Pflaster einen transdermal verabreichbaren Wirkstoff, welcher bevorzugt ausgewählt ist aus der Gruppe bestehend aus Hormonen, Analgetika und Mitteln zur Behandlung neurodegenerativer Erkrankungen. Besonders bevorzugt ist der Wirkstoff ein Mittel zur Behandlung von Morbus Alzheimer.

In einer bevorzugten Ausführungsform ist der Wirkstoff ausgewählt aus der Gruppe bestehend aus Narkotika, Opioiden (einschließlich Opiaten), Tranquilizern, vorzugsweise Benzodiazepinen, Stimulantien und anderen Betäubungsmitteln.

In einer bevorzugten Ausführungsform handelt es sich bei dem Wirkstoff um eine Analgetikum. Bevorzugt werden erfindungsgemäß als Analgetikum diejenigen pharmazeutischen Wirkstoffe verstanden, die von der WHO dem ATC-Index N02 zugeordnet werden (vorzugsweise in der amtlichen deutschen Fassung vom 1. Januar 2005). Bevorzugte Analgetika sind "Opioide" (ATC-Code N02A), "andere Analgetika und Antipyretika" (ATC-Code N02B) und "Migränemittel" (ATC-Code N02C). Innerhalb der bevorzugteren Opioide sind besonders bevorzugt die "natürlichen Opium-Alkaloide" (ATC-Code N02AA), "Phenylpiperidin-Derivate" (ATC-Code N02AB), "Diphenylpropylamin-Derivate" (ATC-Code N02AC), "Benzomorphan-Derivate" (ATC-Code N02AD), "Oripävin-Derivate" (ATC-Code N02AE), "Morphinan-Derivate" (ATC-Code N02AF), "Opioide in Kombination mit Spasmolytika" (ATC-Code N02AG) und "andere Opioide" (ATC-Code N02AX).

In einer besonders bevorzugten Ausführungsform ist der Wirkstoff ein Opioid. Als Opioide eignen sich besonders bevorzugt µ-, κ- oder δ-Opioidrezeptor-Agonisten, ganz besonders bevorzugt µ-Opioidrezeptor-Agonisten.

In einer bevorzugten Ausführungsform ist der Wirkstoff ein transdermal verabreichbares Opioid, Tranquilizer oder ein anderes Betäubungsmittel, das ausgewählt ist aus der Gruppe bestehend aus Alfentanil, Allobarbital, Allylprodin, Alphaprodin, Alprazolam, Amfepramon, Amfetamin, Amfetaminil, Amobarbital, Anileridin, Apocodein, Barbital, Benzylmorphin, Bezitramid, Bromazepam, Brotizolam, Buprenorphin, Butobarbital, Butorphanol, Camazepam, Cathin (D-Norpseudoephedrin), Chlordiazepoxid, Clobazam, Clonazepam, Clonitazen, Clorazepat, Clotiazepam, Cloxazolam, Cocain, Codein, Cyclobarbital, Cyclorphan, Cyprenorphin, Delorazepam, Desomorphin, Dextromoramid, Dextropropoxyphen, Dextromethorphan, Dezocin, Diampromid, Diamorphon, Diazepam, Dihydrocodein, Dihydromorphin, Dimenoxadol, Dimepheptanol, Dimethylthiambuten, Dioxaphetylbutyrat, Dipipanon, Dronabinol, Eptazocin, Estazolam, Ethoheptazin, Ethylmethylthiambuten, Ethylloflazepat, Ethylmorphin, Etonitazen, Etorphin, Fencamfamin, Fenetyllin, Fenproporex, Fentanyl, Fludiazepam, Flunitrazepam, Flurazepam, Halazepam, Haloxazolam, Heroin, Hydrocodon, Hydromorphon, Hydroxypethidin, Isomethadon, Hydroxymethylmorphinan, Ketazolam, Ketobemidon, Levacetylmethadol (LAAM)), Levomethadon, Levorphanol, Levophenacylmorphan, Levoxemacin, Lofentanil, Loprazolam, Lorazepam, Lormetazepam, Mazindol, Medazepam, Mefenorex, Meperidin, Meprobamat, Meptazinol, Metazocin, Methylmorphin, Metamfetamin, Methadon, Methaqualon, Methylphenidat, Methylphenobarbital, Methyprylon, Metopon, Midazolam, Modafinil, Morphin, Myrophin, Nabilon, Nalbuphen, Nalorphin, Narcein, Nicomorphin, Nimetazepam, Nitrazepam, Nordazepam, Norlevorphanol, Normethadon, Normorphin, Norpipanon, Opium, Oxazepam, Oxazolam, Oxycodon, Oxymorphon, Papaver somniferum, Papaveretum, Pernolin, Pentazocin, Pentobarbital, Pethidin, Phenadoxon, Phenomorphan, Phenazocin, Phenoperidin, Piminodin, Pholcodein, Phenmetrazin, Phenobarbital, Phentermin, Pinazepam, Pipradrol, Piritramid, Prazepam, Premethadion, Profadol, Proheptazin, Promedol, Properidin, Propoxyphen, Remifentanil, Secbutabarbital, Secobarbital, Sufentanil, Temazepam, Tetrazepam, Tilidin (cis und trans), Tramadol, Triazolam, Vinylbital, (1R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)cyclohexanol, (1R, 2R)-3-(2-Dimethylaminomethylcyclohexyl)-phenol, (1S, 2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1 RS, 3RS, 6RS)-6-Dimethylaminomethyl-1-(3-methoxy-phenyl)-cyclohexan-1,3-diol, 3-(2-Dimethylaminomethyl-1-hydroxy-cyclohexyl)-phenyl 2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxybenzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydnoxy-biphenyl-4-carbonsäure 3-(2-dimethylaminomethyl-1-hydroxy-cyclohexyl)-phenyl ester sowie deren entsprechende Stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt deren Hydrochloride.

Die Verbindungen (1R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)-cyclohexanol oder deren stereoisomere Verbindungen oder deren physiologisch verträgliche Verbindungen, insbesondere deren Hydrochloride, deren Derivate, wie Ester, Ether oder Amide sowie Verfahren zu ihrer Herstellung sind beispielsweise aus EP-A-693 475 bzw. EP-A-780 369 bekannt. Die entsprechenden Beschreibungen werden hiermit als Referenz eingeführt und gelten als Teil der Offenbarung.

Die Wirkstoffe, ggf. in Form ihrer Ether-, Ester- oder Säurederivate, können jeweils als reines Stereoisomer, insbesondere Enantiomer oder Diastereomer, Racemat oder in Form einer Mischung von Stereoisomeren, insbesondere der Enantiomeren und/oder Diastereomeren, in einem beliebigen Mischungsverhältnis, oder jeweils in Form entsprechender physiologisch verträglicher Salze, oder jeweils in Form entsprechender Solvate vorliegen.

Als geeignete physiologisch verträgliche Salze seien beispielhaft genannt die durch Umsetzung der freien Basen des jeweiligen Wirkstoffs mit einer anorganischen oder organischen Säure, vorzugsweise mit Salzsäure, Bromwasserstoffsäure, Schwefelsäure, Phosphorsäure, Methansulfonsäure, Benzolsulfonsäure, p-Toluolsulfonsäure, Kohlensäure, Ameisensäure, Essigsäure, Propionsäure, Oxalsäure, Bernsteinsäure, Weinsäure, Mandelsäure, Fumarsäure, Maleinsäure, Milchsäure, Äpfelsäure, Sebacinsäure, Citronensäure, Ascorbinsäure, Nicotinsäure, Glutaminsäure oder Asparaginsäure erhaltenen Salze.

Als weitere physiologisch verträgliche Salze seien beispielhaft die durch Umsetzung der freien Säuren des jeweiligen Wirkstoffs mit einer geeigneten Base erhaltenen Salze genannt. Beispielhaft seien die Alkalimetallsalze, Erdalkalimetallsalze oder Ammoniumsalze [NHₓR₄₋ₓ]⁺ genannt, worin x = 0, 1, 2, 3 oder 4 ist und R für einen linearen oder verzweigten C₁₋₄-Alkyl-Rest steht.

Insbesondere bevorzugt ist das Opioid Buprenorphin (ATC-Code N02AE01) als freie Base oder in Form eines seiner pharmazeutisch verträglichen Salze, vorzugsweise Buprenorphin Hydrochlorid, Buprenorphin Saccharinat, Buprenorphin Formiat, Buprenorphin Mesylat, Buprenorphin Hydrogencitrat, Buprenorphin Nicotinat oder Buprenorphin Sebacinat.

In einer bevorzugten Ausführungsform des erfindungsgemäßen Systems ist der Wirkstoff in den Pflastern in unterschiedlichen Dosierungen enthalten und/oder aus den Pflastern mit unterschiedlicher Abgaberate freisetzbar.

Bei den wirkstoffhaltigen Pflastern des erfindungsgemäßen Systems handelt es sich vorzugsweise um Pflaster herkömmlichen Aufbaus, d.h. die Pflaster umfassen neben einer Trägerschicht zumindest eine klebefähige Schicht. Bevorzugte Ausführungsformen derartiger wirkstoffhaltiger Pflaster werden nachfolgend beschrieben:
Die klebefähige Schicht der wirkstoffhaltigen Pflaster basiert bevorzugt auf einem druckempfindlichen Klebstoff. Der druckempfindliche Klebstoff enthält bevorzugt wenigstens ein Polymer, vorzugsweise ausgewählt aus der Gruppe bestehend aus Polyacrylaten, Polyvinylethern, Polyvinylalkoholen, Polyisobutylenen, Acrylat-Copolymerisaten, Ethylen-Vinylacetat-Copolymerisaten, Polyurethanen, Styrol-Isopren-Copolymerisaten, Styrol-Butadien-Copolymerisaten, Cellulose-Derivaten, Silikonen, Kautschuken, Harzen und ggf. hydrierten Estern des Kolophoniums.

Geeignete Cellulose-Derivate sind z.B. Ethylcellulose, Propylcellulose, Hydroxyethylcellulose, Hydroxypropylcellulose und Hydroxypropylmethylcellulose.

Geeignete Polyacrylate sind z.B. (Co-)Polymerisate von Acrylaten, Alkylacrylaten, Methacrylaten, und/oder Alkylmethacrylaten, die ggf. mit weiteren ungesättigten Monomeren wie Acrylamid, Dimethylacrylamid, Dimethylaminoethylacrylat, Hydroxyethylacrylat, Hydroxypropylacrylat, Methoxyethylacrylat, Methoxyethylmethacrylat, Acrylnitril und/oder Vinylacetat copolymerisiert sind.

Im Sinne der Beschreibung bedeutet "(meth)acryl" "acryl" und/oder "methacryl".

Besonders geeignete (Meth)acrylsäureester sind solche, die lineare, verzweigte oder cyclische aliphatische C₁-C₂-Substituenten ohne sonstige funktionelle Gruppen tragen. Zu dieser Gruppe zählen insbesondere Methyl(meth)acrylat, Ethyl(meth)-acrylat, Isopropyl(meth)acrylat, n-Butyl(meth)acrylat, Isobutyl(meth)acrylat, sec.-Butyl(meth)acrylat, tert.-Butyl(meth)acrylat, 2-Ethylhexyl(meth)acrylat, Cyclohexyl-(meth)acrylat und Isobornyl(meth)acrylat.

Doch können auch (Meth)acrylsäureester in dem zur Herstellung des Polyacrylats verwendeten Monomerengemisch enthalten sein, die funktionelle Gruppen tragen. Hierunter sind u.a. hydroxylgruppenhaltige Ester zu verstehen, wie 2-Hydroxyethyl-(meth)acrylat und 3-Hydroxypropyl(meth)acrylat. Aber auch Stoffe wie Acrylnitril, Vinylacetat, Methoxyethyl(meth)acrylat, Acrylamid, Dimethylacrylamid, Dimethylaminoethylacrylat etc. können als funktionelle Gruppen enthaltende (Meth)-acrylsäureester verstanden werden. Der Anteil von (Meth)acrylsäureestem, die derartige funktionelle Gruppen enthalten, soll dabei im Monomerengemisch kleiner oder gleich 10 Gew.%, vorzugsweise weniger als 2 Gew.-%, noch bevorzugter weniger als 0,2 Gew.-%, betragen. Besonders bevorzugt ist ein Monomerengemisch, das keine funktionelle Gruppen enthaltende (Meth)acrylsäureester enthält.

Es kann jedoch auch Vinylacetat als Co-Monomer zusammen mit mindestens einem Monomeren aus der Gruppe der (Meth)acrylsäureester zur Herstellung des Polyacrylats verwendet werden. Der Anteil des Vinylacetats in dem zur Herstellung dieses Polyacrylats verwendeten Monomerengemisch sollte unterhalb von 50 Gew.- %, vorzugsweise unterhalb von 25 Gew. -% liegen. Besonders bevorzugt ist ein Vinylacetatgehalt zwischen 0. und 5,0 Gew.-%. Besonders bevorzugt sind Acrylat-Vinylacetat-Copolymerisate, welche beispielsweise unter der Bezeichnung DURO-TAF^{®} im Handel erhältlich sind. Besonders bevorzugt werden diese Copolymerisate in Kombination mit Polyacrylaten, beispielsweise Carbopol^{®}, insbesondere Carbopol^{®} 980, oder mit Hydroxypropylcellulose eingesetzt.

Beispiele für geeignete Harze sind Ester von Glycinen, Glycerin oder Pentaerythrit, Kohlenwassertoffharze und Polyterpene.

Weiterhin eignen sich Silikon-Klebstoffe, wie z.B. ggf. vernetzte Polydimethylsiloxane.

Der druckempfindliche Klebstoff enthält vorzugsweise Klebrigmacher, welche sich strukturell vorzugsweise vom tricyclo-[5.2.1.0^{2,6}]Decan ableiten. Besonders bevorzugt sind *bis*-(Hydroxymethyl)-tricyclo-[5.2.1.0^{2,6}]decan, *bis*-(Aminomethyl)-tricyclo-[5.2.1.0^{2,6}]decan, und *bis*-Formyl-tricyclo-[5.2.1.0^{2,6}]decan, insbesondere die Derivate des tricyclo-[5.2.1.0^{2,6}]Decans, welche die beiden Substituenten in den Positionen 3(4) und 8(9) tragen. Andere geeignete Klebrigmacher sind Derivate von Kohlenhydraten, insbesondere SAIB.

Der druckempfindliche Klebstoff kann weitere Hilfsstoffe enthalten, welche ihn zur Anwendung auf der menschlichen Haut besonders geeignet machen können. Beispiele für solche Hilfsstoffe sind hautpermeationsfördernde Substanzen, Lösungsmittel, Lösungsvermittler, Emulgatoren, Vernetzer, Stabilisatoren, Füllstoffe (z.B. Zinkoxid, Silica) Konservierungsmittel, Farbstoffe, hautglättende Mittel, Duftstoffe, zur Reduzierung oder Verhinderung von Hautirritationen auf die Hautoberfläche übertragbare Verbindungen, Verdickungsmittel, Weichmacher, Klebrigmacher, etc. Solche Hilfsstoffe sind dem Fachmann bekannt. In diesem Zusammenhang kann beispielsweise verwiesen werden auf H.P. Fiedler, Lexikon der Hilfsstoffe für Pharmazie, Kosmetik und angrenzende Gebiete, Editio Cantor Aulendorff, 2002; R. Niedner et al., Dermatika: therapeutischer Einsatz, Pharmakologie und Pharmazie, Wiss. Verl.-Ges. 1992. Konkrete Beispiele für die vorstehend genannten Substanzklassen werden nachfolgend auch im Zusammenhang mit weiteren Aspekten der Erfindung beschrieben.

Der Wirkstoff kann in der klebefähigen Schicht (*drug in adhesive*) und/oder einer weiteren Schicht, beispielsweise in einer von der klebefähigen Schicht separierten Matrix (*drug in matrix*), enthalten sein.

In einer bevorzugten Ausführungsform ist der Wirkstoff wenigstens teilweise in der klebefähigen Schicht enthalten. In einer bevorzugten Ausführungsform ist der Wirkstoff wenigstens teilweise in einer Matrix eingebettet, wobei die Matrix ihrerseits Bestandteil der klebefähigen Schicht sein oder eine eigenständige Schicht bilden kann.

In einer bevorzugten Ausführungsform handelt es sich um ein Matrix-Pflaster. Das Matrix-Pflaster umfasst bevorzugt eine Trägerschicht, von der eine Oberfläche an die Matrix angrenzt, welche den Wirkstoff enthält (wirkstoffhaltige Schicht), und eine klebefähige Schicht. Die klebefähige Schicht bedeckt vorzugsweise die gesamte Fläche der Trägerschicht. Die dazwischen liegende Matrix bedeckt vorzugsweise nur einen Teil der Fläche der Trägerschicht, so dass ein äußerer Rand der Trägerschicht verbleibt, welcher zwar mit der klebefähigen Schicht, nicht jedoch mit der Matrix bedeckt ist. Um die Migration des Wirkstoffs in die Trägerschicht zu minimieren, kann es vorteilhaft sein, zwischen Trägerschicht und Matrix eine Sperrschicht aus einem geeigneten Material einzufügen.

Bei dem Matrix-Pflaster ist der Wirkstoff zumindest zum Teil in einer Matrix eingebettet (wirkstoffhaltige Schicht). Die Matrix basiert vorzugsweise auf lipophilen oder hydrophilen, vorzugsweise vernetzbaren Polymeren. Hydrophile matrixbildende Schichten können wasserhaltig sein, wobei es sich in einem solchen Fall vorzugsweise um Gele handelt. Vorzugsweise basiert die Matrix auf wenigstens einem Polymer ausgewählt aus der Gruppe umfassend Cellulose-Derivate, wie z.B. Cellulose-Ether, besonders bevorzugt Methylcellulose, Ethylcellulose, Propylcellulose, Hydroxyethylcellulose, Hydroxypropylcellulose, Hydroxypropylmethylcellulose, und/oder Carboxymethylcellulose; Polyethylene; chlorierte Polyethylene, wie Polyvinylchloride oder Polyvinylidenchloride; Polypropylene; Polyurethane; Polycarbonate; Polyacrylsäureester; Polyacrylate; Polymethacrylate; Polyvinylalkohole; Polyvinylpyrrolidone; Polyethylenterephthalate; Polytetrafluorethylene; Ethylen-Propylen-Copolymere; Ethylen-Ethylacrylat-Copolymere; Ethylen-Vinylacetat-Copolymere; Ethylen-Vinylalkohol-Copolymere; Ethylen-Vinyloxyethanol-Copolymere; Vinylchlorid-Vinylacetat-Copolymere; Vinylpyrrolidon-Ethylen-Vinylacetat-Copolymere; Kautschuke; synthetische Homo-, Co- oder Blockpolymere aus Butadien und/oder Styrol, wie Styrol-Isopren-Styrol-Blockcopolymere; Silikone; Silkon-Derivate, besonders bevorzugt Siloxan-Methacrylat-Copolymere, Polyvinylether, Polyester, und/oder Polysaccharide, bzw. deren Mischungen. Besonders bevorzugt sind Polyacrylate oder Acrylat Copolymerisate, insbesondere solche, wie sie vorstehend im Zusammenhang mit den Polymeren des druckempfindlichen Klebstoffs beschrieben sind.

In einer weiteren bevorzugten Ausführungsform handelt es sich bei dem wirkstoffhaltigen Pflaster um ein sog. "monolithisches *drug-in-adhesive*" Pflaster. In diesem Fall ist der Wirkstoff in der klebefähigen Schicht enthalten (*drug in adhesive*)*.* Ein derartiges Pflaster umfasst bevorzugt eine Trägerschicht und eine klebefähige Schicht, welche den Wirkstoff enthält. Die wirkstoffhaltige Schicht kann zusätzlich matrixbildende Polymere enthalten, so dass der Wirkstoff in einer Matrix eingebettet ist (*drug in matrix*), welche ihrerseits Bestandteil der klebefähigen Schicht ist (*drug in adhesive*). Zur Herstellung einer solchen klebefähigen Schicht können die Bestandteile des druckempfindlichen Klebstoffs als klebefähige Komponente mit den vorstehend aufgeführten Matrixmaterialien in bekannten Mengen gemischt werden und zur Herstellung des wirkstoffhaltigen Matrixbereichs eine physiologisch wirksame Substanz hinzugefügt werden. Nach dem Auftrag kann das Matrixmaterial, wenn nötig, auch noch vernetzt werden. Der in der Matrix vorliegende Wirkstoff oder die in der Matrix vorliegenden Wirkstoffe können flüssig, halbfest oder fest im dispergierten Zustand vorliegen oder als entsprechende Formulierung unter Hinzufügung von üblichen Hilfsstoffen als Wirkstoffformulierung eingearbeitet sein.

Sofern das wirkstoffhaltige Pflaster keine vollflächige klebefähige Schicht aufweist, kann es auch so gestaltet sein, dass der druckempfindliche Klebstoff nur in den Randzonen der wirkstoffhaltigen Schicht vorliegt, wobei diese Randzonen vorzugsweise keinen Wirkstoff enthalten.

In einer weiteren bevorzugten Ausführungsform ist das wirkstoffhaltige Pflaster mehrschichtig, wobei der Wirkstoff zumindest zum Teil in der klebefähigen Schicht enthalten ist. Bei dieser Ausführungsform ist wenigstens eine selektiv-permeable Membran zwischen zwei klebefähigen Schichten vorhanden, wobei beide klebefähigen Schichten wirkstoffhaltig sind (vgl. T.A. Petersen et al., Intern. Symp. Control. Rel. Bioact. Mater., 21:477-478). Geeignete selektiv-permeable Membranen sind kommerziell erhältlich (vgl. z.B. R.E. Kesting, Synthetic Polymer Membranes, McGraw Hill; J.D. Ferry, Ultrafiltration Membranes, Chemical Review, 18, 373).

In einer anderen bevorzugten Ausführungsform handelt es sich bei dem wirkstoffhaltigen Pflaster um ein Reservoir-Pflaster. Das Reservoir-Pflaster umfasst vorzugsweise eine Trägerschicht und ein Reservoir, welches den Wirkstoff in gelöster oder suspendierter Form enthält, wobei das Reservoir zumindest auf der Seite, welche bei der Applikation der Haut zugewandt ist, mit einer selektiv-permeablen Membran umgeben ist. Die Trägerschicht weist vorzugsweise eine gewisse mechanische Beständigkeit und ggf. Seitenwände auf. Die Kapazität des Reservoirs ist von der räumlichen Ausdehnung des so gebildeten Hohlraums abhängig. Die klebefähige Schicht kann sich über die gesamte Fläche des Reservoir-Pflasters, welche bei der Applikation der Haut zugewandt ist, erstrecken. Es ist aber auch möglich, dass nur Teile dieser Fläche mit der klebefähigen Schicht bedeckt sind, etwa nur die der Haut zugewandte Fläche der Trägerschicht, welche nicht mit dem Reservoir bzw. der selektiv-permeablen Membran bedeckt ist.

Handelt es sich um ein Reservoir-Pflaster, so liegt der Wirkstoff in einem Reservoir vor. Das Reservoir wird vorzugsweise von einer selektiv-permeablen Membran umschlossen oder zumindest auf einer Seite von dieser begrenzt. Als Membranmaterialien sind die vorstehend aufgeführten Polymere geeignet, die auch als Matrixmaterial zum Einsatz kommen können. Vorzugsweise basiert die Membran auf wenigstens einem Polymer ausgewählt aus der Gruppe bestehend aus Polyethylenen, Polypropylenen, Polyvinylacetaten, Polyamiden, Ethylen-Vinylacetat-Copolymeren, Polyethylenterephthalaten und Silikonen. Mit Hilfe der Reservoir-Membran kann aus dem Reservoir eine kontrollierte Freisetzung des Wirkstoffes erzielt werden.

Das Reservoir kann vorzugsweise zwischen einer Deckschicht und der klebefähigen Schicht angeordnet sein. Das Reservoir kann ggf. auch als ein Vlies, Gewebe oder dergleichen ausgebildet sein, welches mit der Wirkstoff-Formulierung getränkt ist.

Das Reservoir enthält wenigstens einen Wirkstoff, vorzugsweise als Lösung, die durch die das Reservoir umschließende Membran ungehindert diffundieren kann. Die Lösung kann eine erhöhte Viskosität aufweisen, beispielsweise kann es sich um eine Hydrogel oder um ein Lipogel handeln. Ein Hydrogel enthält vornehmlich hydrophile Substanzen, jedoch nicht notwendigerweise Wasser. Als Lösungsmittel sind solche Lösungsmittel geeignet, in denen sich der Wirkstoff ausreichend löst, so dass dadurch eine Ausfällung der Wirkstoffe vermieden wird.

Sind die wirkstoffhaltigen Pflastern zur transdermalen Verabreichung des enthaltenen Wirsktoffs bestimmt, so können als übliche Hilfsstoffe Verbindungen eingesetzt werden, die den transdermalen Transport der Wirkstoffe verstärken bzw. erleichtern (hautpermeationsfördernde Substanzen). Es können die dem Fachmann für den jeweiligen, transdermal zu verabreichenden Wirkstoff bekannten hautpermeationsfördernden Substanzen verwendet werden. In diesem Zusammenhang kann beispielsweise verwiesen werden auf D.S. Hsieh, Drug Permeation Enhancement: Theory and Applications (Drugs and the Pharmaceutical Sciences: a Series of Textbooks and Monographs), Marcel Dekker, 1994 und E.W. Smith et al., Percutaneous Penetration Enhancers, CRC Press, 1995.

Ferner kann die wirkstoffhaltige Schicht - ggf. neben matrixbildenden Polymeren und/oder hautpermeationsverbessemden Substanzen - übliche Hilfsstoffe enthalten, wie z.B. Lösungsmittel, Lösungsvermittler, Emulgatoren, Vernetzer, Stabilisatoren, Konservierungsmittel, Weichmacher, Farbstoffe, hautglättende Mittel, Duftstoffe und/oder Verdickungsmittel. Zur Erzeugung ph-kontrollierter Bedingungen auf der Haut können als Hilfsstoffe z.B. schwache Säuren, schwache Basen oder (an)organische Salze, welche ein Puffersystem auf der Haut bilden, zugegeben werden. Ferner ist der Zusatz von Kristallisationsinhibitoren oder hochdispersem Siliciumdioxid in einer penetrationsfördernden Menge möglich. Diese Hilfsstoffe können mit den Wirkstoffen gemischt oder in einer separaten Schicht zur wirkstoffhaltigen Schicht vorliegen.

Sämtliche Materialien, die zum Aufbau der transdermalen Systeme verwendet werden, insbesondere aber diejenigen Materialien, die mit der Haut in Berührung kommen, sollten hautverträglich und physiologisch unbedenklich sein.

Die Schichtdicke der klebefähigen Schicht des wirkstoffhaltigen Pflasters beträgt vorzugsweise 3 bis 100 µm, bevorzugt 5 bis 90 µm und insbesondere 10 bis 80 µm.

Das wirkstoffhaltige Pflaster hat vorzugsweise eine Gesamtfläche (Kontaktfläche zur Haut bei bestimmungsgemäßer Anwendung) von 0,5 bis 200 cm², bevorzugter 1,0 bis 175 cm², noch bevorzugter 20 bis 150 cm². In einer bevorzugten Ausführungsform hat das wirkstoffhaltige Pflaster eine Gesamtfläche von 0,5 bis 100 cm², bevorzugter 0,75 bis 75 cm², noch bevorzugter 1,0 bis 50 cm², am bevorzugtesten 1,5 bis 50 cm² und insbesondere 2,0 bis 25 cm².

Bei dem wirkstoffhaltigen Pflaster grenzt die klebefähige Schicht vorzugsweise unmittelbar an eine Trägerschicht an.

Die Trägerschicht umfasst vorzugsweise ein flexibles, dehnbares, atmungsaktives, strapazierfähiges Material und kann gefärbt, beispielsweise hautfarben sein. Die Trägerschicht weist vorzugsweise eine solche Dicke auf, dass das wirkstoffhaltige Pflaster eine ausreichende mechanische Stabilität aufweist. Vorzugsweise ist die Trägerschicht wirkstoffundurchlässig, d.h. zumindest in dem Bereich, in dem die Trägerschicht an eine wirkstoffhaltige Matrix bzw. ein Wirkstoffreservoir angrenzt, ist sie für den Wirkstoff undurchlässig. Ist die Trägerschicht als solche nicht wirkstoffundurchlässig, sollte zwischen der wirkstoffhaltigen Schicht, d.h. der Matrix bzw. dem Wirkstoffreservoir, und der Trägerschicht eine wirkstoffundurchlässige Sperrschicht angeordnet sein. Diese Sperrschicht basiert dann vorzugsweise gleich oder verschieden auf einem oder mehreren Materialien, wie nachfolgend für die Trägerschicht ausgeführt:
Als Beispiele für geeignete Materialien der Trägerschicht können insbesondere genannt werden Polyester bzw. Copolyester, bevorzugt Polyethylenterephthalate; Polyolefine bzw. Olefin-Copolymerisate, bevorzugt Polyethylene, Polypropylene oder Polybutylene; Polycarbonate; Polyethylenoxide; Polyurethane; Polystyrole; Polyamide bzw. Copolyamide; Polyimide; Polyvinylacetate; Polyvinylchloride; Polyvinylidenchloride; Copolymerisate, bevorzugt Acrylnitril-Butadien-Styrol-Terpolymere oder Ethylen-Vinylacetat-Copolymerisate, Papier, Textilien und deren Mischungen. Besonders bevorzugt basiert die Trägerschicht auf einem Polyester, insbesondere auf einem Polyethylenterephthalat, beispielsweise auf Hostaphan^{®} RN. Die Trägerschicht kann auch metallisiert und/oder pigmentiert oder als eine Schichtfolge aus den vorstehend genannten Materialien und einer Metallfolie, besonders bevorzugt einer Folie aus Aluminium, bestehen.

In einer bevorzugten Ausführungsform ist die Trägerschicht mit einem anorganischorganischen Hybridpolymer beschichtet. Anorganisch-organische Hybridpolymere, sogenannte Ormocere, sind im Stand der Technik bekannt (vgl. z.B. EP-A 0 358 011; EP-A 0 373 451; EP-A 0 610 831; EP-B 0 644 908; EP-A 0 792846; EP-A 0 934 989).

In einer bevorzugten Ausführungsform weist die Trägerschicht eine Schichtdicke im Bereich von 5,0 bis 125 µm, bevorzugter 10 bis 115 µm noch bevorzugter 25 bis 100 µm, am bevorzugtesten 35 bis 95 µm und insbesondere 50 bis 85 µm auf.

Bevorzugt bildet die Trägerschicht eine der beiden Oberflächenschichten des wirkstoffhaltigen Pflasters, d.h. ausgehend von der vorzugsweise unmittelbar an die Trägerschicht angrenzenden klebefähige Schicht enthält das wirkstoffhaltige Pflaster jenseits der Trägerschicht bevorzugt keine weitere Schicht.

In einer weiteren bevorzugten Ausführungsform umfasst das wirkstoffhaltige Pflaster mehrere vereinzelbare Untereinheiten, welche ihrerseits in Form einer eigenständigen Einheit als wirkstoffhaltiges Pflaster verwendet werden können, wobei die einzelnen Untereinheiten miteinander über vorzugsweise wirkstofffreie Randbereiche, in denen Trennungsmöglichkeiten vorgesehen sind, zu einer zusammenhängenden Einheit verbunden sind. Vorzugsweise liegen linienförmige Perforationen zur Trennung der vereinzelbaren Untereinheiten von der zusammenhängenden Einheit vor. Weiter bevorzugt sind Schneidemarkierungen auf der Trägerschicht, welche die Trennmöglichkeit der vereinzelbaren Untereinheiten von der Einheit vorgeben. Die Trennmöglichkeiten sind jeweils vorzugsweise so angeordnet, dass sie eine Abtrennung jeder zusammenhängenden Untereinheit ermöglichen. Die einzelnen Untereinheiten können gleich oder verschieden sein. Sind die Untereinheiten verschieden, so können sie beispielsweise den darin enthaltenen Wirkstoff in verschiedenen Dosierungen enthalten bzw. mit verschiedenen Freisetzungsraten abgeben.

Die Herstellung des wirkstoffhaltigen Pflasters kann nach bekannten Herstellungsverfahren erfolgen und übliche Verfahrensschritte umfassen, wie Laminieren, Coextrudieren, Stanzen, Delaminieren, Abwickeln, Schneiden, Wiederaufwickeln, Montieren oder Dosieren (Verpackungs-Rundschau 4/2002, 83-84).

Ein weiterer Gegenstand der Erfindung betrifft eine Verpackung, welche das vorstehend beschriebene, erfindungsgemäße System umfasst.

In einer bevorzugten Ausführungsform ist dabei die gemeinsame Schutzfolie mit den daran haftenden wirkstoffhaltigen Pflastern aus der Verpackung herausnehmbar.

In einer anderen bevorzugten Ausführungsform ist die gemeinsame Schutzfolie nicht aus der Verpackung herausnehmbar, sondern ist entweder fest mit zumindest einer Außenwand der Verpackung verbunden oder bildet ihrerseits zumindest eine Außenwand der Verpackung. Bei dieser Variante haften die Pflaster auf der gleichen, dem Innenraum der Verpackung zugewandten Oberflächenseite der gemeinsamen Schutzfolie. Die andere Oberflächenseite der gemeinsamen Schutzfolie bildet zumindest eine Außenseite der Verpackung.

Diese Ausführungsform hat den Vorteil, dass die gemeinsame Schutzfolie nicht innerhalb der Verpackung verkanten kann, was ein Herausnehmen eventuell erschwerten würde. Darüber hinaus kann sich ein "*cold flow*" des Materials der klebefähigen Schicht nicht negativ auswirken. So ist bekannt, dass das Material der klebefähigen Schicht mitunter ein gewisses Fließverhalten zeigt (sog. "*cold flow*").

Dieses Fließverhalten ist in der Regel zwar nur gering ausgeprägt, kann bei Lagerung der Pflaster über mehrere Monate jedoch sehr wohl in Erscheinung treten und bei herkömmlichen Verpackungen Probleme bereiten. Das Fließverhalten hat zur Folge, dass das Material der klebefähigen Schicht mit der Zeit seitlich am Rand der Pflaster austritt, dadurch in Kontakt mit der Verpackung kommt und daran anhaftet. Wird eine herkömmliche Verpackung an ihrer Stirnseite geöffnet, so dass ein einseitig geöffneter Beutel entsteht, so kann die in der Verpackung liegende, gemeinsame Schutzfolie infolge der Anhaftung nicht lose entnommen werden, sondern es muss zunächst aktiv die unbeabsichtigte, auf den "*cold flow*" zurückzuführende Fixierung gelöst werden. Insbesondere ältere oder behinderte Menschen haben dann Probleme, die gemeinsame Schutzfolie aus der Verpackung zu entnehmen.

Wird hingegen erfindungsgemäß die gemeinsame Schutzfolie in die Verpackung integriert, d.h. haften die Pflaster auf der gleichen Oberflächenseite der gemeinsamen Schutzfolie und bildet die andere Oberflächenseite der gemeinsamen Schutzfolie zumindest eine Außenseite der Verpackung, so wird durch Öffnen der Verpackung die gesamte gemeinsame Schutzfolie freigelegt und ein Verkanten oder Anhaften der gemeinsamen Schutzfolie innerhalb der Verpackung verhindert. Auf diese Weise wird also die Handhabbarkeit der Verpackung verbessert. Bei dieser Ausführungsform ist die Verpackung vorzugsweise als Aufreißverpackung ausgebildet, welche beim bestimmungsgemäßen Öffnen wie ein Buch aufgeklappt wird. Die eine Hälfte der aufgeklappten Verpackung wird dann von der gemeinsamen Schutzfolie mit den daran anhaftenden Pflastern gebildet, die andere Hälfte wird von dem Verpackungsteil gebildet, welcher im verschlossenen Zustand der Verpackung deckungsgleich darüber lag.

In einer bevorzugten Ausführungsform ist die erfindungsgemäße Verpackung wiederverschließbar. Realisierungsmöglichkeiten wiederverschließbarer Verpackungen sind dem Fachmann bekannt. Bevorzugte Realisierungsmöglichkeiten sind selbstklebende Flächen and den zur Öffnung der Verpackung vorgesehenen Stellen oder auch *zip-lock* Mechanismen. In diesem Zusammenhang kann beispielsweise verwiesen werden auf EP-A 1 590 260, EP-A 1 500 601, EP-A 1 366 999, EP-A 1 338 525, EP-A 1 448 387 und EP-A 1 513 730.

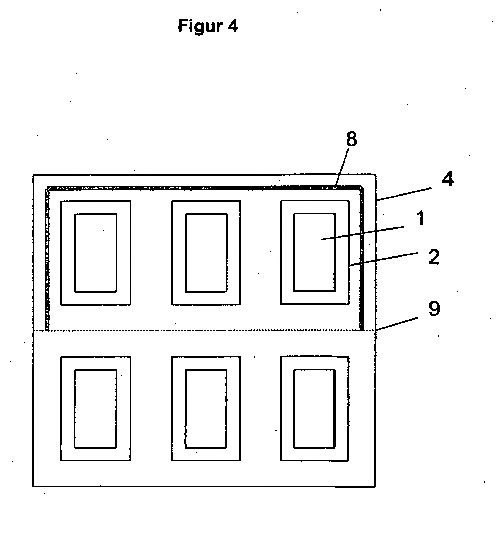

Figur 4 zeigt schematisch eine bevorzugte Ausführungsform einer wiederverschließbaren, erfindungsgemäßen Verpackung. Dabei sind sechs Pflaster (1) jeweils von einer vorzugsweise transparenten Abdeckfolie (2) bedeckt. Die Pflaster (1) haften über ihre klebefähige Schicht an einer gemeinsamen Schutzfolie (4). Die gemeinsame Schutzfolie (4) weist am oberen Rand einen selbstklebenden Bereich (8) auf. Wird die gemeinsame Schutzfolie (4) über die Falz (9) zusammengeklappt, so kann die Verpackung mit Hilfe des selbstklebenden Bereichs (8) verschlossen werden.

## Patentansprüche

1. System umfassend mehrere wirkstoffhaltige Pflaster (1) und eine gemeinsame Schutzfolie (4), wobei die Pflaster (1) jeweils eine klebefähige Schicht aufweisen, welche auf einem druckempfindlichen Klebstoff basiert, der zur Haftung des wirkstoffhaltigen Pflasters (1) auf der Haut vorgesehen ist, über diese klebefähige Schicht an der gemeinsamen Schutzfolie (4) haften und davon ablösbar sind, **dadurch gekennzeichnet, dass**
jedes Pflaster (1) jeweils von einer Abdeckfolie, (2) bedeckt ist, deren Fläche größer als die Fläche des Pflasters (1) ist, wobei die Abdeckfolie (2) in den Randbereichen (3) ihrer dem Pflaster (1) zugewandten Oberflächenseite ablösbar mit der gemeinsamen Schutzfolie (4) verbunden ist, so dass die Abdeckfolie (2) zusammen mit der gemeinsamen Schutzfolie (4) einen geschlossenen Hohlraum (5) bildet, in dem das Pflaster (1) angeordnet ist, und wobei die Abdeckfolie (2) vollflächig selbstklebend ausgerüstet ist und auf der Oberflächenseite des wirkstoffhaltigen Pflasters (1), welche der klebefähigen Schicht abgewandt ist, haftet, so dass folgende Wechselwirkungen über selbstklebende Bereiche vorliegen:
(i) Haftung der klebefähigen Schicht des wirkstoffhaltigen Pflasters (1) auf der gemeinsamen Schutzfolie (4);
(ii) Haftung der selbstklebenden Abdeckfolie (2) auf der Rückseite des wirkstoffhaltigen Pflasters (1), welche der klebefähigen Schicht des wirkstoffhaltigen Pflasters (1) abgewandt ist, und
(iii) Haftung der selbstklebenden Abdeckfolie (2) auf der gemeinsamen Schutzfolie (4) im Randbereich (3) der Abdeckfolie (2);
wobei die Klebkraft der Haftung (i) und die Klebkraft der Haftung (iii) jeweils kleiner als die Klebkraft der Haftung (ii) ist.

2. System nach Anspruch 1, **dadurch gekennzeichnet, dass** die Abdeckfolie (2) einen transparenten Bereich aufweist.

3. System nach Anspruch 1 oder 2, **dadurch gekennzeichnet, dass** die Pflaster (1) einen transdermal verabreichbaren Wirkstoff enthalten.

4. System nach Anspruch 3, **dadurch gekennzeichnet, dass** der Wirkstoff ausgewählt ist aus Hormonen, Analgetika und Mitteln zur Behandlung neurodegenerativer Erkrankungen.

5. System nach einem der vorstehenden Ansprüche, **dadurch gekennzeichnet, dass** der Wirkstoff in den Pflastern (1) in unterschiedlichen Dosierungen enthalten ist und/oder aus den pflastern (1) mit unterschiedlicher Abgaberate freisetzbar ist.

6. System nach einem der vorstehenden Ansprüche, **dadurch gekennzeichnet, dass** die Pflaster (1) gleiche oder verschiedene Markierungen (6) aufweisen.

7. System nach Anspruch 6, **dadurch gekennzeichnet, dass** die Pflaster (1) in Gruppen eingeteilt werden können, wobei sich Pflaster (1) der gleichen Gruppe durch gleiche Markierungen (6) und Pflaster (1) verschiedener Gruppen durch verschiedene Markierungen (6) auszeichnen.

8. System nach Anspruch 6 oder 7, **dadurch gekennzeichnet, dass** die Schutzfolie (4) für jedes Pflaster (1) eine der Markierung (6) des jeweiligen Pflasters (1) entsprechende oder davon verschiedene Markierung (6) aufweist.

9. System nach Anspruch 8, **dadurch gekennzeichnet, dass** die Markierungen (6) der gemeinsamen Schutzfolie (4) für ein bestimmtes Pflaster (1) erst dann nach außen sichtbar wird, wenn dieses Pflaster (1) von der gemeinsamen Schutzfolie (4) abgelöst wird.

10. System nach einem der vorstehenden Ansprüche, **dadurch gekennzeichnet, dass** sie eine Vorrichtung (7) aufweist, um die Pflaster (1) nach deren bestimmungsgemäßer Anwendung aufzunehmen.

11. Verpackung umfassend ein System nach einem der Ansprüche 1 bis 10.

12. Verpackung nach Anspruch 11, **dadurch gekennzeichnet, dass** die gemeinsame Schutzfolie (4) aus der Verpackung herausnehmbar ist.

13. Verpackung nach Anspruch 11, **dadurch gekennzeichnet, dass** die Pflaster (1) auf der gleichen Oberflächenseite der Schutzfolie (4) haften und die andere Oberflächenseite der Schutzfolie (4) eine Außenseite der Verpackung bildet.

14. Verpackung nach einem der Ansprüche 11 bis 13, **dadurch gekennzeichnet, dass** sie wiederverschließbar ist.

## Claims

1. System comprising a plurality of active-substance-containing plasters (1) and a common protective film (4), the plasters (1) each having an adhesive layer based on a pressure-sensitive adhesive provided for adhering the active-substance-containing plaster (1) to the skin, adhering to the common protective film (4) via said adhesive layer and being detachable therefrom, **characterised in that** each plaster (1) is covered by a cover film (2), of which the surface area is greater than the surface area of the plaster (1), the cover film (2) being detachably connected to the common protective film (4) in the edge regions (3) of its ' surface side facing the plaster (1), in such a way that the cover film (2), together with the common protective film (4), forms a closed cavity (5), in which the plaster (1) is arranged, and the cover film (2) being self-adhesive over its entire surface and adhering to the surface side of the active-substance-containing plaster (1) remote from the adhesive layer, in such a way that the following interactions occur over the self-adhesive regions:
(i) adhesion of the adhesive layer of the active-substance-containing plaster (1) to the common protective film (4);
(ii) adhesion of the self-adhesive cover film (2) to the rear face of the active-substance-containing plaster (1) remote from the adhesive layer of the active-substance-containing plaster (1), and
(iii) adhesion of the self-adhesive cover film (2) to the common protective film (4) in the
edge region (3) of the cover film (2);
the adhesive force of the adhesion (i) and the adhesive force of the adhesion (iii) each being less than the adhesive force of the adhesion (ii).

2. System according to claim 1, **characterised in that** the cover film (2) comprises a transparent region.

3. System according to either claim 1 to claim 2, **characterised in that** the plasters (1) contain an active substance that can be administered transdermally.

4. System according to claim 3, **characterised in that** the active substance is selected from hormones, analgesics and drugs for treating neurodegenerative diseases.

5. System according to any one of the preceding claims, **characterised in that** the active substance is contained in the plasters (1) in different doses and/or can be released from the plasters (1) at different delivery rates.

6. System according to any one of the preceding claims, **characterised in that** the plasters (1) comprise the same or different markings (6).

7. System according to claim 6, **characterised in that** the plasters (1) can be divided into groups, the plasters (1) in the same group being distinguished by the same markings (6) and the plasters (1) in different groups being distinguished by different markings (6).

8. System according to either claim 6 or claim 7, **characterised in that** the protective film (4) for each plaster (1) comprises a marking (6) corresponding to or different from the marking (6) of the respective plaster (1).

9. System according to claim 8, **characterised in that** the markings (6) of the common protective film (4) for a specific plaster (1) are only externally visible when this plaster (1) is detached from the common protective film (4).

10. System according to any one of the preceding claims, **characterised in that** it comprises a device (7) for collecting the plasters (1) following their intended application.

11. Packaging comprising a system according to any one of claims 1 to 10.

12. Packaging according to claim 11, **characterised in that** the common protective film (4) can be removed from the packaging.

13. Packaging according to claim 11, **characterised in that** the plasters (1) adhere to the same surface side of the protective film (4) and the other surface side of the protective film (4) forms an outer face of the packaging.

14. Packaging according to any one of claims 11 to 13, **characterised in that** it is resealable.

## Revendications

1. Système comprenant plusieurs pansements (1) contenant une/des substances actives et une feuille de protection commune (4), où les pansements (1) présentent à chaque fois une couche adhésive qui est à base d'un adhésif sensible à la pression qui est prévue pour l'adhérence du pansement (1) contenant une/des substances actives sur la peau, adhèrent via cette couche adhésive sur la couche de protection commune (4) et sont amovibles de celle-ci, **caractérisé en ce que** chaque pansement (1) est à chaque fois recouvert par une feuille de recouvrement (2), dont la surface est supérieure à la surface du pansement (1), où la feuille de recouvrement (2) est assemblée de manière amovible au niveau des zones de bord (3) de sa face orientée vers le pansement (1) avec la feuille de protection commune (4), de manière telle que la feuille de recouvrement (2) forme ensemble avec la feuille de protection commune (4) un espace creux fermé (5), dans lequel le pansement (1) est disposé et où la feuille de recouvrement (2) est apprêtée de manière autoadhésive sur toute sa surface et adhère sur la face du pansement (1) contenant une/des substances actives qui est opposée à la couche adhésive, de manière telle que les interactions suivantes sont présentes via des zones autoadhésives :
(i) adhérence de la couche adhésive du pansement (1) contenant une/des substances actives sur la feuille de protection commune (4) ;
(ii) adhérence de la feuille de recouvrement (2) autoadhésive sur la face arrière du pansement (1) contenant une/des substances actives, qui est opposée à la couche adhésive du pansement (1) contenant une/des substances actives et
(iii) adhérence de la feuille de recouvrement autoadhésive (2) sur la feuille de protection commune (4) au niveau de la zone de bord (3) de la feuille de recouvrement (2) ;
l'adhésivité de l'adhérence (i) et l'adhésivité de l'adhérence (iii) étant à chaque fois inférieures à l'adhésivité de l'adhérence (ii).

2. Système selon la revendication 1, **caractérisé en ce que** la feuille de recouvrement (2) présente une zone transparente.

3. Système selon la revendication 1 ou 2, **caractérisé en ce que** les pansements (1) contiennent une substance active pouvant être administrée par voie transdermique.

4. Système selon la revendication 3, **caractérisé en ce que** la substance active est choisie parmi les hormones, les analgésiques et les agents pour le traitement de maladies de neurodégénérescence.

5. Système selon l'une quelconque des revendications précédentes, **caractérisé en ce que** la substance active est contenue dans les pansements (1) en des dosages différents et/ou est libérable des pansements (1) avec une vitesse de libération différente.

6. Système selon l'une quelconque des revendications précédentes, **caractérisé en ce que** les pansements (1) présentent des marquages identiques ou différents (6).

7. Système selon la revendication 6, **caractérisé en ce que** les pansements (1) peuvent être répartis en groupes, où les pansements (1) du même groupe se caractérisent par des marquages (6) identiques et les pansements (1) de groupes différents se caractérisent par des marquages (6) différents.

8. Système selon la revendication 6 ou 7, **caractérisé en ce que** la feuille de protection (4) présente, pour chaque pansement (1), un marquage (6) correspondant au ou différent du marquage (6) de ce pansement (1).

9. Système selon la revendication 8, **caractérisé en ce que** le marquage (6) de la feuille de protection commune (4) pour un pansement (1) donné ne devient visible vers l'extérieur que lorsque ce pansement (1) est détaché de la feuille de protection commune (4).

10. Système selon l'une quelconque des revendications précédentes, **caractérisé en ce qu'**il présente un dispositif (7) destiné à recevoir le pansement (1) après son utilisation conforme à sa destination.

11. Emballage comprenant un système selon l'une quelconque des revendications 1 à 10.

12. Emballage selon la revendication 11, **caractérisé en ce que** la feuille de protection commune (4) peut être enlevée de l'emballage.

13. Emballage selon la revendication 11, **caractérisé en ce que** les pansements (1) adhèrent sur la même face de la feuille de protection (4) et l'autre face de la feuille de protection (4) forme une face extérieure de l'emballage.

14. Emballage selon l'une quelconque des revendications 11 à 13, **caractérisé en ce qu'**il est refermable.
